(19)

Europäisches
Patentamt
European
Patent Office
Office européen
des brevets

(11) **EP 2 503 339 A1**

(12) **EUROPEAN PATENT APPLICATION**

(43) Date of publication:
**26.09.2012 Bulletin 2012/39**

(51) Int Cl.:
*G01N 33/68* (2006.01)    *G01N 33/92* (2006.01)

(21) Application number: **11159462.8**

(22) Date of filing: **23.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Nederlandse Organisatie voor Toegepast -Natuurwetenschappelijk Onderzoek TNO 2628 VK Delft (NL)**

(72) Inventor: **The designation of the inventor has not yet been filed**

(74) Representative: **Jansen, Cornelis Marinus et al VEREENIGDE Johan de Wittlaan 7 2517 JR Den Haag (NL)**

(54) **Improved prediction of cardiovascular and metabolic disease development**

(57)     The invention relates to a method to improve the risk analysis of a subject for developing a cardiovascular disease or a (pre-)diabetic condition, comprising including in the risk analysis lipoprotein metabolic ratios derived from the lipoprotein profile of said subject. The lipoprotein metabolic rations are preferably derived from the Particle Profiler system, which is extensively described in the present invention.

EP 2 503 339 A1

**Description**

FIELD OF THE INVENTION

The invention relates to the field of diagnostics, especially medical diagnostics. More particularly, the invention is related to the prediction of susceptibility to cardiovascular and metabolic diseases, such as diabetes, insulin resistance, metabolic syndrome, atherosclerosis, heart failure, stroke, etc. Especially, the invention is related to finding new diagnostic markers derived from the lipoprotein profile.

BACKGROUND

Cardiovascular and metabolic diseases often are partly caused by or accompanied by aberrations from the normal lipid metabolism. Especially serum low-density lipoprotein (LDL) cholesterol and LDL particle concentrations are known to be positively associated with cardiovascular disease risk (Cromwell, W.C. and Otvos, J.D, 2004, Curr. Atheroscl. Rep. 6:381-387), and reaching low LDL cholesterol concentrations is a primary goal for therapy (Grundy, S.M. et al., 2004, Circulation, 110:227-239).

Technological advances allow the size spectrum of lipoproteins to be measured in increasing detail, creating a detailed lipoprotein profile (e.g. Caulfield, M. et al., 2008, Clin. Chem. 54:2088-2089). The detailed lipoprotein profile needs further interpretation and validation to be useful in the clinic. One example of interpreting this detailed data is the pooling of all LDL particles, and reporting an 'LDL particle number'. This diagnostic has proven to be successful at predicting cardiovascular risk (Cromwell and Otvos, *supra).* However, the detailed lipoprotein profiles contain more information that is discarded when only reporting LDL particles. A computational model that can characterize the state of metabolic processes affecting lipoproteins, based on the additional information contained in a lipoprotein profile, may be of added value in the clinic.

Lipoprotein metabolism of VLDL, IDL and LDL comprises three main processes. The lipoproteins are produced by the liver, then lose triglycerides through lipolysis and are finally taken up from the bloodstream by the liver. The lipolysis process occurs in extrahepatic tissues through lipoprotein lipase (LPL), which mainly affects the larger very-low-density lipoproteins (VLDL) (Olivecrona, T. et al., 1997, Proc. Nutr. Soc. 56:723-729), whereas in the liver lipolysis occurs through hepatic lipase (HL), mainly affecting the smaller IDL and LDL (Demant, T. et al., 1988, J. Lipid Res. 29: 1603-1611). Measuring the rates of these processes is generally carried out using stable-isotope or radioactive-isotope tracer techniques. The most popular approaches perform kinetic tracer analysis of the large constituent protein apolipoprotein B to obtain lipoprotein fluxes (Parhofer, K. and Barrett, P., 2006, J. Lipid Res. 47:1620-1630). These techniques are costly and labor-intensive. A good characterization of the status of lipoprotein metabolism is, therefore, an extensive and difficult procedure at this time.

Thus, there is need for an easy-to-use lipoprotein characterization in order to assist with the diagnosis and or prediction of diabetes and/or cardiovascular disease.

SUMMARY OF THE INVENTION

Recently, a new computational model of lipoprotein metabolism ('Particle Profiler') was developed by the current inventor (van Schalkwijk, D. et al., 2009, J. Lipid Res. 50:2398-2411; WO 2009/151324). It appears that it is possible to use this model to derive parameter values that can be used to improve the prediction of risk for metabolic and cardiovascular diseases.

Therefore, the current invention relates to a method to improve the risk analysis of a subject for developing a cardiovascular disease or a (pre-)diabetic condition, comprising including in the risk analysis lipoprotein metabolic ratios derived from the lipoprotein profile of said subject. Preferably, the risk analysis is performed via a multivariate analysis. Further preferred, the disease is a cardiovascular disease, preferably a disease selected from the group of atrial fibrillation, (congestive) heart failure, coronary heart disease, intermittent claudication, stenosis, thromboembolism, myocardial infarction, coronary insufficiency, angina, ischemic stroke, hemorrhagic stroke, ischemia, and peripheral artery disease.

More particularly, the risk analysis is based on one or more of the classic parameters age of the subject, sex of the subject, systolic and/or diastolic blood pressure, body-mass index, smoking behaviour, serum cholesterol concentration and serum glucose concentration. In such a method, the lipoprotein metabolic ratios from the lipoprotein profile included in the risk

analysis are selected from the group of: $\ln\left(\dfrac{J_{\text{in},ILDL}}{k_{\text{a,liver}}^{ILDL}}\right)$ or parameters that are

highly correlated therewith as indicated in Table 2; $\ln\left(\dfrac{J_{in,ILDL}}{k_{a,liver}^{ILDL}}\right)$ or parameters

that are highly correlated therewith as indicated in Table 2; $\ln\left(\dfrac{J_{in,ILDL}}{k_1^{ILDL}}\right)$ or parameters that are highly correlated

therewith as indicated in Table 2; $\ln\left(\dfrac{\overline{k_1^{ILDL}}}{k_{u,liver}^{ILDL}}\right)$ or parameters that are highly correlated therewith as indicated in Table

2; $\ln\left(\dfrac{\overline{k_{a,liver}^{ILDL}}}{k_1^{ILDL}}\right)$ or parameters that are highly correlated therewith as indicated in Table 2; or $\ln\left(\dfrac{\overline{k_{hl}^{ILDL}}}{k_1^{ILDL}}\right)$ or parameters

that are highly correlated therewith as indicated in Table 2; and any combinations thereof, and preferably at least two, preferably at least three, more preferably at least four and most preferably at least five of the lipoprotein metabolic ratios are included in the risk analysis.

In another embodiment, the present invention relates to a method according to the invention, wherein the disease is a (pre-)diabetic condition, preferably a disease selected from the group of diabetes, in particular diabetes type 2, insulin resistance, impaired glucose tolerance, metabolic syndrome, and obesitas. Preferably, therein the risk analysis is based on one or more of the classic parameters: sex of the subject, systolic and/or diastolic blood pressure, body-mass index, smoking behaviour of the spouse, serum HDL cholesterol concentration and serum glucose concentration and, optional, the HDL particle number and the VDL particle number. In this embodiment, the lipoprotein metabolic ratios from the lipoprotein profile included in the risk analysis are selected from the group of: $\ln\left(\dfrac{J_{in,ILDL}}{k_{u,liver}^{ILDL}}\right)$ or parameters that are highly correlated therewith as indicated in Table 3; $\ln\left(\dfrac{\overline{k_{lpl}^{ILDL}}}{k_{a,liver}^{ILDL}}\right)$ or parameters that

are highly correlated therewith as indicated in Table 3; $\ln\left(\dfrac{\overline{k_{hl}^{ILDL}}}{k_1^{ILDL}}\right)$ or

parameters that are highly correlated therewith as indicated in Table 3; and any combinations thereof.

Further, the subject in any method according to the invention preferably is a human being.

Also part of the invention is the use of lipoprotein metabolic rations from the lipoprotein profile of a subject for improving the risk analysis of a subject for developing a cardiovascular disease or a (pre-)diabetic condition.

LEGENDS TO THE FIGURES

**Figures 1-9. Step-by-step explanation of model development for liver-lipolysis and uptake**

For explanation, see text.

**Figure 10. ROC curves of the cross-validated multivariate models for general cardiovascular disease, for the datasets indicated in table 6 of example 4.**

**Figure 11. Data use and generation during model development and model implementation.**

During model development, Particle Profiler was used as indicated below the vertical bar: pooled lipoprotein flux data was used for fitting the model to data of individual subjects, and the fitted model was used to generate lipoprotein particle flux data and lipoprotein metabolic ratios. The light blue area illustrates the usage of the model in its application for generating diagnostic markers for cardiovascular disease and diabetes. In that application, Particle Profiler was applied to lipoprotein profile data, allowing the quantification of lipoprotein metabolic ratios that were used as diagnostic markers.

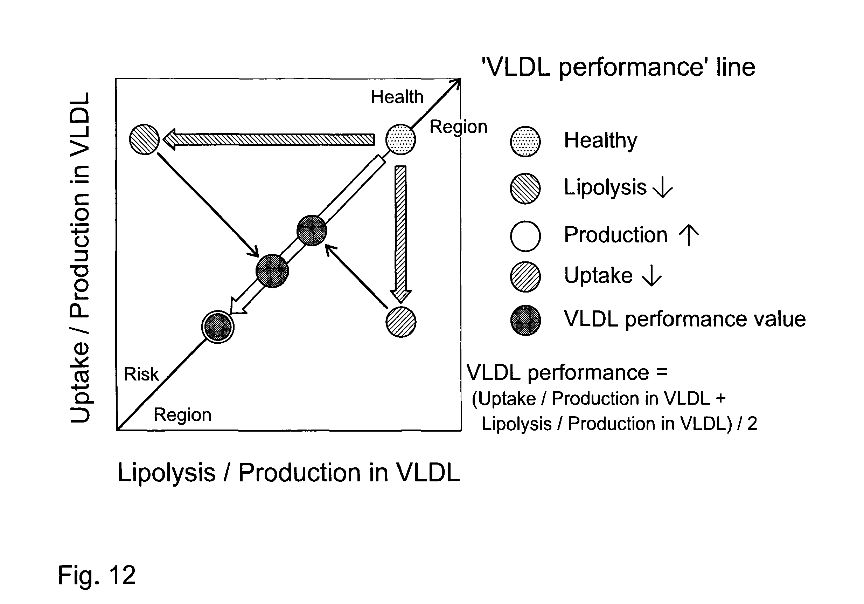

**Figure 12 - Graphical representation of the VLDL Performance diagnostic.**

When applying the Particle Profiler model to a lipoprotein profile, the uptake / production and lipolysis / production ratios in VLDL was quantified. The information from these ratios was summarized in a single statistic taking the mean of these two ratios, which was visualized as a projection on the identity line. We propose the name VLDL Performance for this projection. It integrates information about production, lipolysis and uptake rates, but can be calculated without metabolic flux information, based on one detailed lipoprotein profile measured in one fasting blood sample.

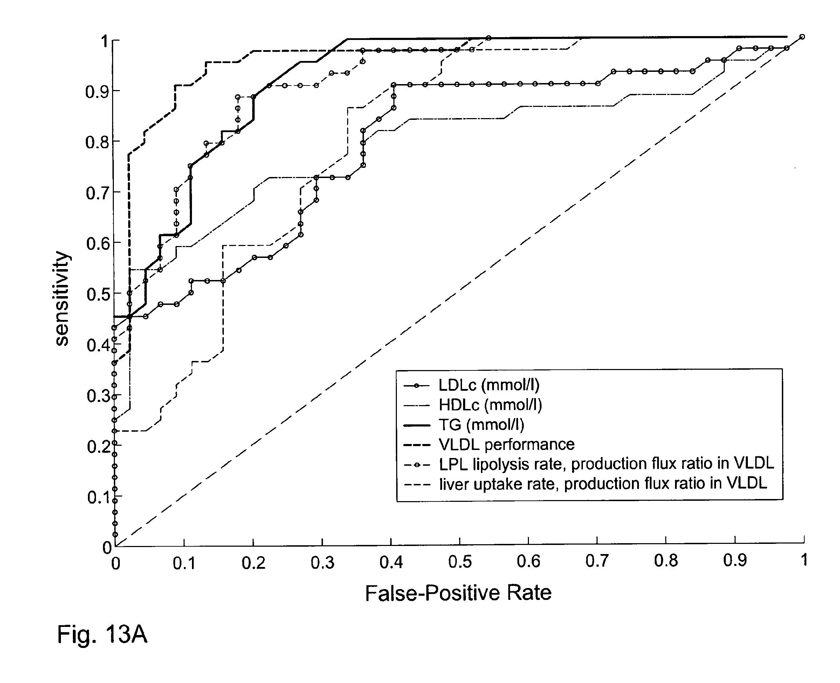

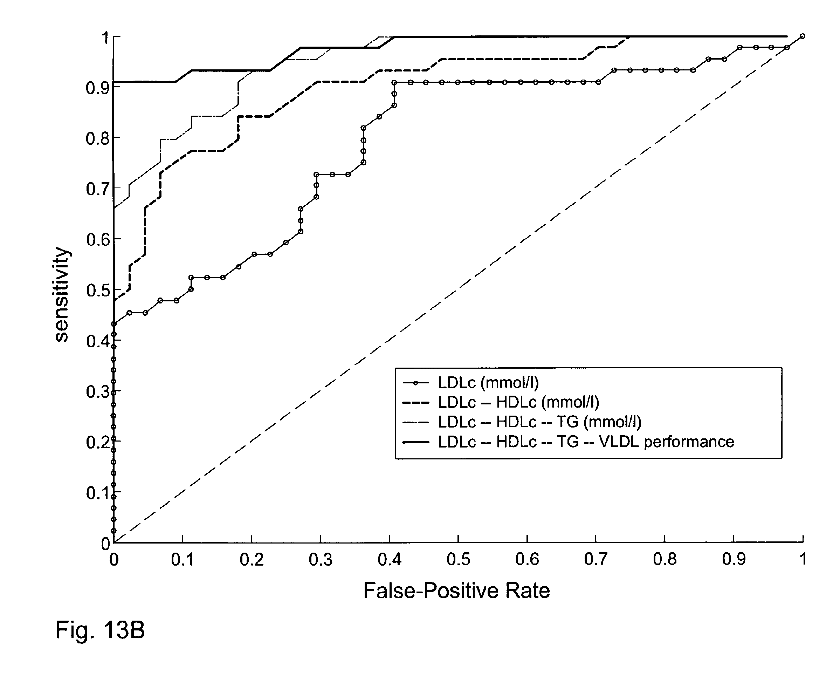

**Figure 13 - Receiver operating characteristic (ROC) curves for dyslipidemia**

These curves indicate how well a) single diagnostics and b) multivariate regression models distinguish dyslipidemic subjects from normolipidemic subjects reported in a range of studies described in the text under example 3. The models in b) subsequently include LDLc, HDLc, TG and VLDL performance in cumulative fashion. This ROC curve indicates the sensitivity of the indicated diagnostics for identifying dyslipidemic subjects, when varying the acceptable false-positive rate. The further away from the 1-1 identity line, the better the diagnostic. For example, when not accepting false positives, the regression model including LDLc, HDLc and TG has a sensitivity of 66% (0.66), while additionally including the VLDL Performance diagnostic results in a sensitivity of 91% (0.91).

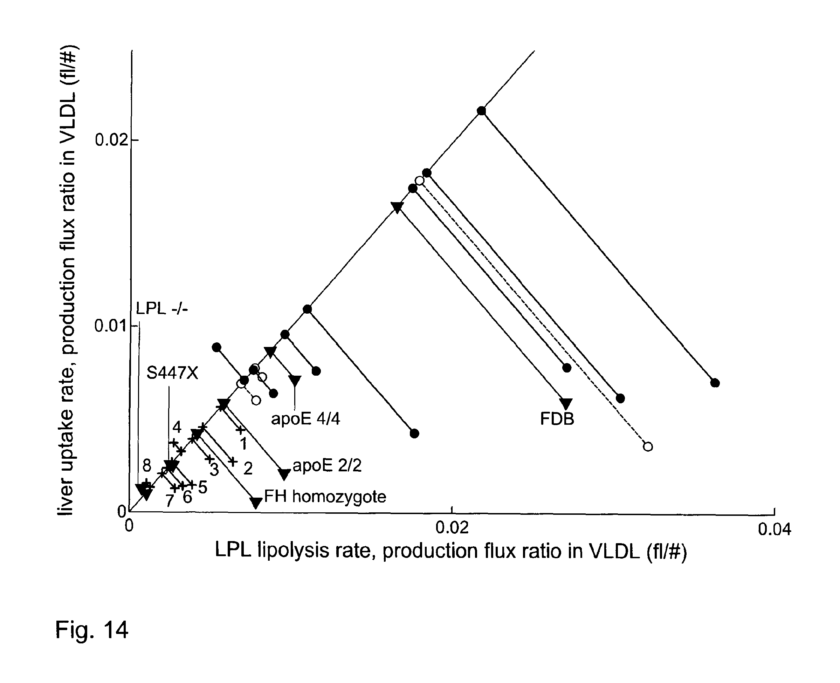

**Figure 14 - The average VLDL Performance of various subject groups**

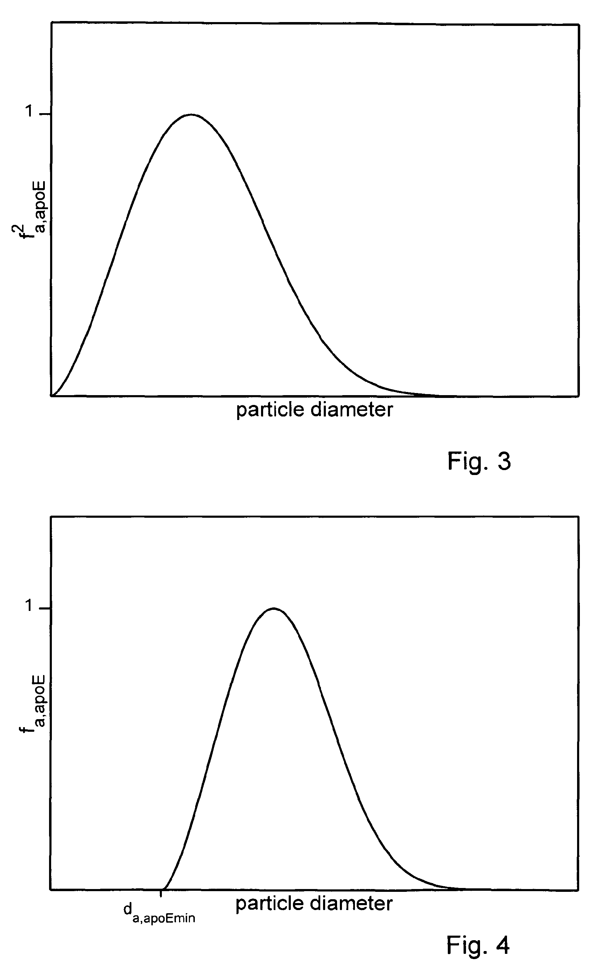

Green lines with round ends are normolipidemic subject groups. Groups indicated with darker green were used for the ROC curve in figure 3, those indicated with light green were not. Red lines with crosses represent dyslipidemic subject groups used for the ROC curve. Groups are labeled as follows: 1) hypothyroid patients during T4 treatment; 2) subjects with small LDL peak size; 3 and 8) mixed hyperlipidemia; 4) hypothyroid patients before treatment; 5)kidney disease: membranous glomerulonephritis; 6) patients on HIV treatment; 7) kidney disease: focal segmental glomerulosclerosis. Blue lines with triangles indicate subject groups with specific genetic variant.. FDB: Familial Defective ApoB ; FH: Familial Hypercholesterolemia ; S447X: specific single nucleotide polymorphism in the LPL gene. The data from these subjects was taken from the studies mentioned in the text under examples 2 and 3.

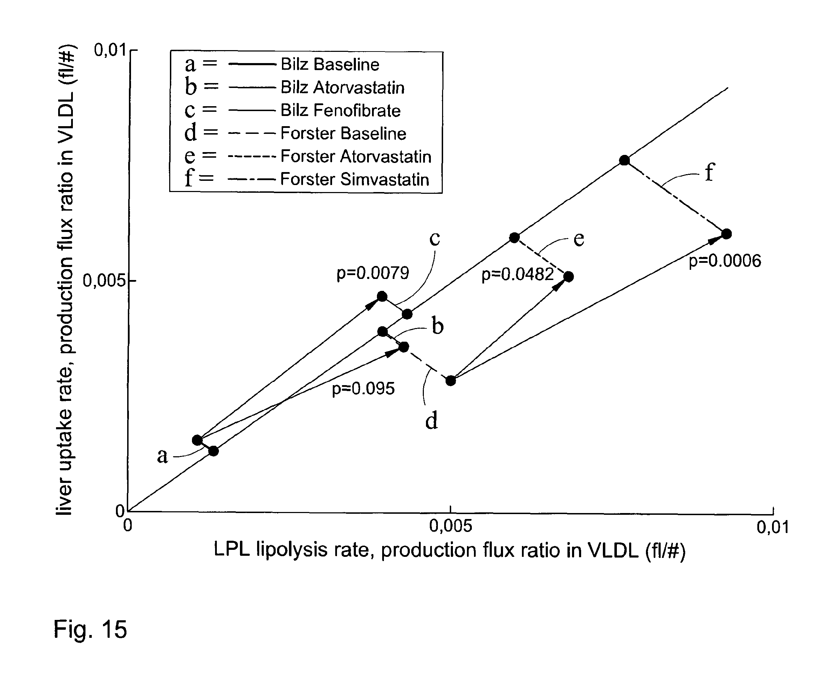

**Figure 15 - VLDL Performance response to treatment**

Average VLDL performance response (on identity line) to atorvastatin, simvastatin and fenofibrate treatment in mixed hyperlipidemic patients. P-values for VLDL performance were calculated by the Wilcoxon rank-sum test. Bilz et al.(2004, J. Lipid Res. 45 :174-185) atorvastatin: n=5, p=0.0925; Bilz fenofibrate: n=5, p=0.0079; Forster et al. (2002, Atherosclerosis 164:129-145) atorvastatin: n=9, p=0.0482; Forster simvastatin: n=11, p=0.0006. All treatments caused VLDL performance to move towards healthier values.

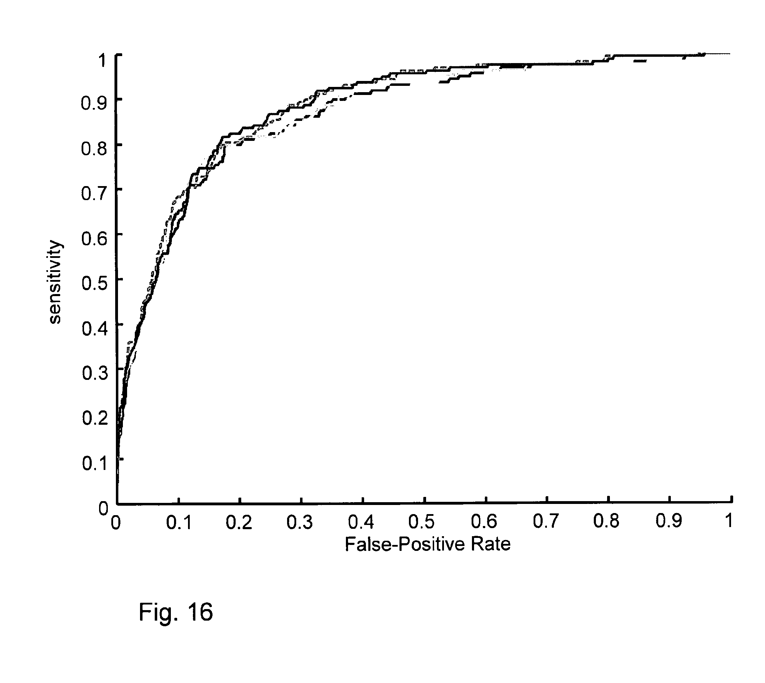

**Figure 16 - ROC curves of the diabetes risk analysis**

ROC curves of the cross-validated multivariate models for the classic (black), classic & NMR (very light grey), Classic & NMR & ratios(1) (dotted grey), Classic & NMR & ratios (2) (grey) datasets.

DETAILED DESCRIPTION OF THE INVENTION

The computational model of lipoprotein metabolism called 'Particle Profiler' has been extensively described in van Schalkwijk et al., 2009 (*supra*) and WO 2009/151324. The Particle Profiler model describes how lipoprotein production, lipolysis and uptake processes depend on lipoprotein size. Equations for this size dependence cannot be derived mechanistically, since biological knowledge is not sufficiently detailed to allow for such a derivation. Biological knowledge does, however, provide clues as to how these functions should look in a general sense. These clues are mentioned in the 'biology' paragraph van Schalkwijk et al., 2009. and their interpretation is mentioned in the 'Conceptual Model' paragraph of each modelled process in the same article. Based on this information the Particle Profiler equations were motivated. How this motivation translated into the Particle Profiler equations has been explained in the additional material of the article.

The Particle Profiler model as described in WO 2009/151324 and van Schalkwijk et al., 2009, was used with some modifications, as described below. The description and working example of the model as given on page 9, line 29, to page 29, line 10, of WO 2009/151324 is included herein by reference.

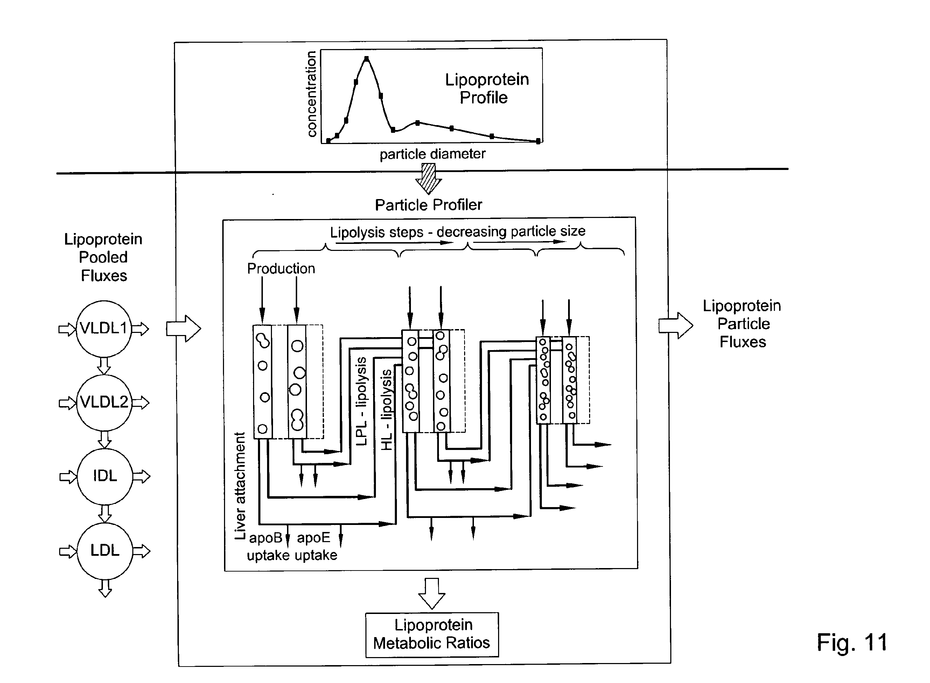

In the Particle Profiler model, the metabolic rate of a particle depends on its size. Figure 11 shows how in the model development phase, reported in the current invention, we chose to derive particle-based lipoprotein fluxes and lipoprotein metabolic ratios from previously published pooled lipoprotein flux data. This 'pooled lipoprotein flux data' includes particle concentrations and fluxes (production, lipolysis and uptake) in four size classes: VLDL1, VLDL2, IDL and LDL. Figure 11 also shows that the application of Particle Profiler to lipoprotein profiles, as reported in the current invention under Example 4, is not able to produce particle-based lipoprotein fluxes, but only lipoprotein metabolic ratios. It is impossible to obtain the absolute fluxes, since the lipoprotein profile measurements are taken from a single blood sample and do not contain explicit kinetic information. Still, the metabolic ratios show whether metabolic processes are well balanced or dysbalanced. Nevertheless, the lipoprotein metabolic ratios can advantageously be applied in the present invention.

In the model development phase, when using pooled lipoprotein flux data as input, the Particle Profiler model can calculate the average metabolic rate of particles in a certain size range of interest, for instance the VLDL size range. The model also distinguishes different metabolic routes, such as particle lipolysis through LPL or HL. It is impossible to measure these quantities directly, even with tracers. Instead, a feasible approach to technical validation is to calibrate the model with pooled lipoprotein flux data from genetically deficient subjects, and subsequently corroborate it with pooled lipoprotein flux data from a range of different normolipidemic and dyslipidemic subjects. Calibration and subsequent corroboration with pooled lipoprotein flux data is the only available route of technical validation.

The mathematical functions describing liver uptake and lipolysis needed further development compared with the original Particle Profiler model (as described in van Schalkwijk et al., 2009 (*supra*) and WO 2009/151324) for two reasons. First of all, for three out of sixteen previously analyzed subjects, the model was not able to reproduce the lipoprotein fluxes well. The deviation was mainly due to the uptake fluxes, suggesting that the mathematical functions used to model uptake processes were suboptimal. Secondly, a parameter identifiability analysis, using the covariance matrix produced by the parameter fitting routine (data not shown), showed that detailed lipoprotein profiles, in contrast to lipoprotein kinetics data, did not contain enough information to fit the six parameters in the original model. Because of problems for future model applications to lipoprotein profile data, we decided to reduce the dimensionality of the model by one parameter to five parameters through simplifying the hepatic lipolysis function. Since the two mentioned issues relate to liver-related processes, we introduced new functions for lipoprotein attachment to the liver, and lipoprotein lipolysis and uptake by the liver.

The new model of liver-related aspects of lipoprotein metabolism describes the biological process as two phases, similar to the earlier model. In the first phase, the particle is attached to the liver, via either apoB- or apoE-related mechanisms. In the second phase, particles attached through the apoB-related mechanism are directly taken up, whereas particles attached through the apoE-related mechanism can be either taken up or lipolyzed. The probability that a particle is taken up or lipolyzed depends on the size of the particle, with larger particles having a greater probability of being taken up instead of lipolyzed.

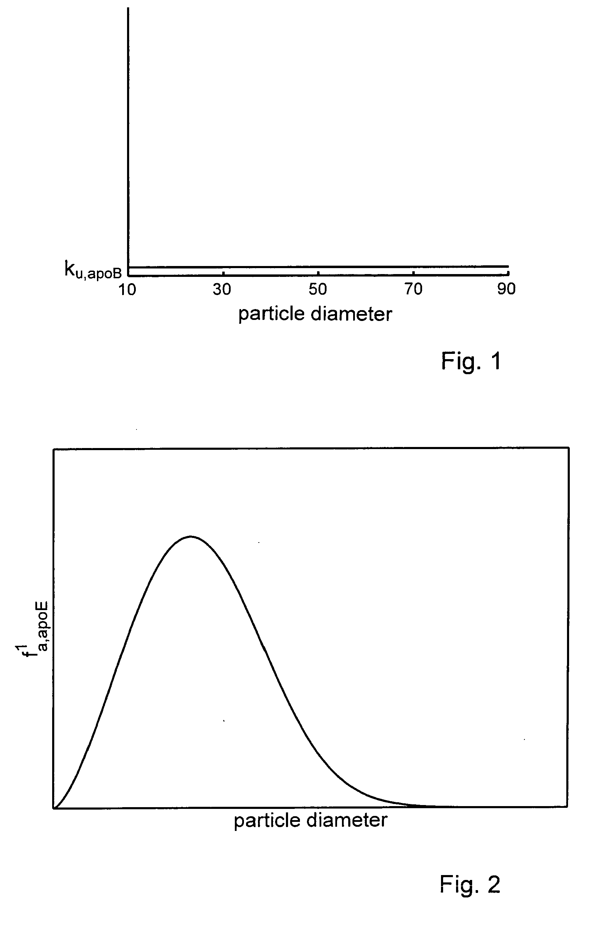

In our model particles can be processed by the liver in two ways. They can be directly taken up via an apoB-related mechanism. They can also be attached to the liver via an apoE-related mechanism, which can result either in particle uptake or lipolysis. The apoB-related uptake mechanism is modeled to have the same rate at all particle sizes, which therefore always equals $k_{u,apoB}$. (see Fig. 1)

For apoE-related attachment to the liver we expect a pattern which first increases and then decreases with particle size. Furthermore, the initial increase should be gradual, so that the liver can gradually increase the lipolysis of smaller particles. The probability density function of the single parameter Rayleigh distribution we initially used (van Schalkwijk et al., 2009), met the first requirement, but not the second. Therefore, we now switched to the more general Weibull distribution, and constructed a suitable one-parameter version of this distribution. This Weibull distribution is not interpreted as a probability distribution, but it rather describes the apoE related attachment rate at different particle sizes as a fraction ($f_{a,apoE}(d)$) of the maximum apoE related attachment rate (see Fig. 2). This leads to the following first candidate model:

$$f_{a,apoE}^1 (d) = BA^{-B} d^{B-1} e^{-\left(\frac{d}{A}\right)^B}$$

eq. 1

One problem of the equation in this form is that its maximum value does not equal one. This is undesirable since we would like to introduce a parameter which represents the maximum liver attachment rate. This maximum rate should be reached at the peak of the modeled distribution (Fig. 3). Therefore we now first scale the Weibull probability density function with its maximum value, which can vary according to the parameter values. We can calculate the value of d at which this maximum is reached by equating the derivative of $f_{a,apoE}^1$ to zero. We find that:

$$\frac{d}{dd} f_{a,apoE}^1 \left( e^{\left( \frac{\ln\left(\frac{B-1}{B}\right)}{B} \right)} \cdot A \right) = 0$$

eq. 2

By scaling the Weibull distribution with its maximum, its peak value always equals one. The second candidate model thus becomes:

$$f_{a,apoE}^2(d) = \left( \frac{f_{a,apoE}^1(d)}{f_{a,apoE}^1 \left( e^{\left( \frac{\ln\left(\frac{B-1}{B}\right)}{B} \right)} \cdot A \right)} \right) = d^{B-1} \frac{e^{-\left(\frac{d}{A}\right)^B}}{e^{\left( \ln\left(\frac{B-1}{B}\right)-1 \right)\left(\frac{B-1}{B}\right)} \cdot A^{B-1}}$$

eq. 3

Next, the equation needs to be shifted horizontally to start at the smallest particle size at which liver attachment occurs (Fig. 4). The final model for the fraction of maximum apoE-related uptake thus becomes:

$$f_{a,apoE}(d) = \begin{cases} (d - d_{a,apoE\min})^{B-1} \dfrac{e^{\frac{-(d-d_{a,apoE\min})^B}{A}}}{e^{\left( \ln\left(\frac{B-1}{B}\right)-1 \right)\left(\frac{B-1}{B}\right)} \cdot A^{B-1}} & for \quad d \geq d_{a,apoE\min} \\ 0 & for \quad d < d_{a,apoE\min} \end{cases}$$

eq. 4

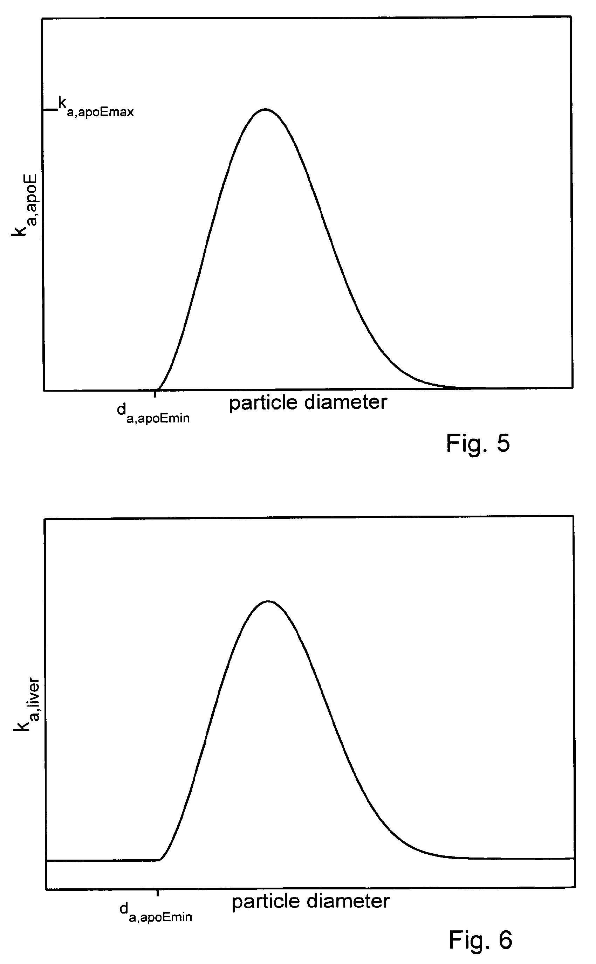

We can now calculate the apoE related attachment rate, by introducing a parameter representing the maximum liver attachment rate of the apoE-related process. (Fig. 5)

$$k_{a,apoE}(d) = \begin{cases} k_{a,apoE\max} \left( (d - d_{a,apoE\min})^{B-1} \dfrac{e^{\frac{-(d-d_{a,apoE\min})^B}{A}}}{e^{\left( \ln\left(\frac{B-1}{B}\right)-1 \right)\left(\frac{B-1}{B}\right)} \cdot A^{B-1}} \right) & for \quad d \geq d_{a,apoE\min} \\ 0 & for \quad d < d_{a,apoE\min} \end{cases}$$

eq.5

Since liver attachment includes an apoB-related and an apoE-related contribution (Fig. 6), we calculate the total liver attachment rate as follows (eq. 6):

$$k_{a,liver}(d) = \begin{cases} k_{a,apoE\max} \left( (d - d_{a,apoE\min})^{B-1} \dfrac{e^{\frac{-(d-d_{a,apoE\min})^B}{A}}}{e^{\left( \ln\left(\frac{B-1}{B}\right)-1 \right)\left(\frac{B-1}{B}\right)} \cdot A^{B-1}} \right) + k_{a,apoB} & for \quad d \geq d_{a,apoE\min} \\ k_{a,apoB} & for \quad d < d_{a,apoE\min} \end{cases}$$

In the apoB-related mechanism liver attachment is immediately followed by uptake. The apoE-related attachment mechanism can either be followed by lipolysis or uptake. The model for this last process does not include a pool of attached particles. The model rather considers that attachment is directly followed by either uptake or lipolysis. This is obviously a simplification, but a necessary one since data are not available to parameterize a more detailed process. Because of this simplification we can state in terms of fluxes ($J$) that:

$$J_{a,liver}(d) = J_{l,liver}(d) + J_{u,liver}(d) \qquad \textit{eq. 6}$$

which can be rewritten in terms of pools (*Q-dimension: # of particles*) and rate constants (*k-dimension: 1/t*) to the following:

$$Q(d) \cdot k_{a,liver}(d) = Q(d) \cdot k_{l,liver}(d) + Q(d) \cdot k_{u,liver}(d) \qquad \textit{eq. 7}$$

and by dividing away the pools we get:

$$k_{a,liver}(d) = k_{l,liver}(d) + k_{u,liver}(d) \qquad \textit{eq. 8}$$

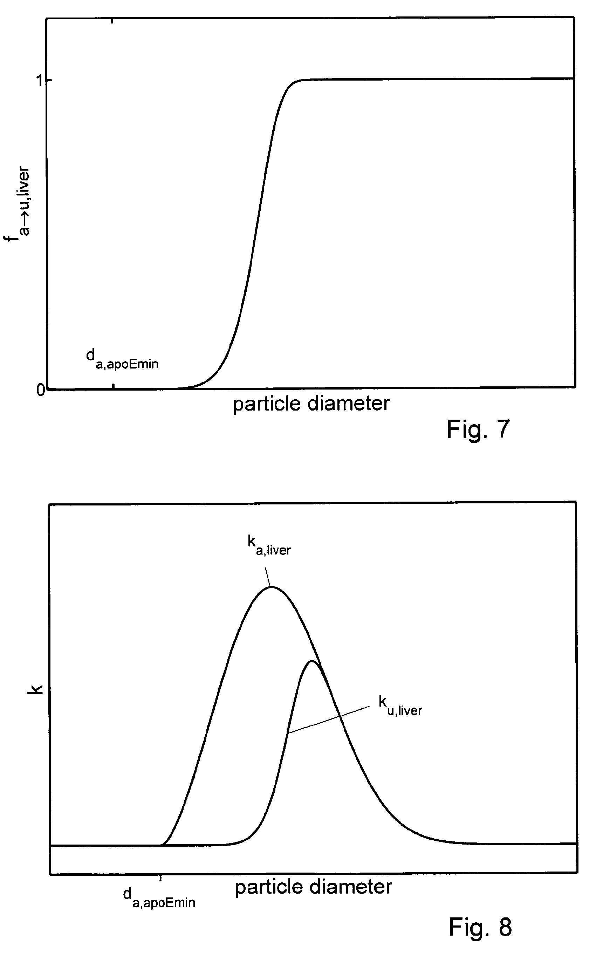

In order to model how the ratio between uptake and lipolysis in the liver depends on particle size, we apply the consideration that hepatic lipase mainly acts on smaller particles. This means that the fraction of particles taken up after liver attachment increases with increasing particle size (Fig. 7). To model this process we use the cumulative probability density function of the Weibull distribution, leading to the following equation for the fraction of attached particles that is taken up by the liver instead of lipolysed ($f_{a \to u,liver}$):

$$f_{a \to u,liver}(d) = \begin{cases} 1 - e^{-s_{u,liver}^{\frac{1}{2}}\left(\frac{d - d_{a,apoE\ min}}{\sigma_{u,liver}}\right)} & \textit{for} \quad d \geq d_{a,apoE\ min} \\ 0 & \textit{for} \quad d < d_{a,apoE\ min} \end{cases} \qquad \textit{eq. 9}$$

Now, we also need to take into account that in our model hepatic uptake consists of two contributions. The first is that of apoB-related uptake, which is constant irrespective of particle size. The second is that of apoE-related uptake, which does depend on particle size (Fig. 8). In the final equation for liver uptake we combine the contributions of both processes:

$$k_{u,liver}(d) = \begin{cases} \left(k_{a,liver} - k_{a,apoB}\right)\left(1 - e^{-s_{u,liver}^{\frac{1}{2}}\left(\frac{d - d_{a,apoE min}}{\sigma_{u,liver}}\right)}\right) + k_{a,apoB} & \textit{for} \quad d \geq d_{a,apoE min} \\ k_{a,apoB} & \textit{for} \quad d < d_{a,apoE min} \end{cases} \qquad \textit{eq.10}$$

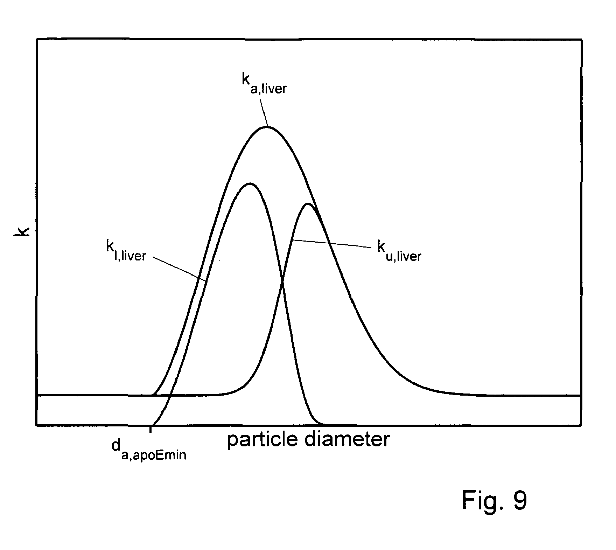

using the relation between hepatic attachment, hepatic uptake and hepatic lipolysis motivated above, we can now calculate hepatic lipolysis as follows (Fig. 9):

$$k_{l,liver}(d) = k_{a,liver}(d) - k_{u,liver}(d) \qquad \textit{eq.11}$$

**Model Calibration**

The model equations contain model parameters that are allowed to assume different values for different patients and model constants that are fixed to the same value for all patients. The model constants contain the biological information that, for instance, allows the model to distinguish hepatic lipolysis from extrahepatic lipolysis. Therefore, it is very important that these constants have the correct values. The constants optimized here are: $d_{hl,peak}$ $s_{u,liver}$ σ $_{a,lpl}$ and $d_{a,lp\,min}$, which are related to HL lipolysis, liver uptake and LPL lipolysis (2 constants) respectively (see Table 1 for an overview of all notations). The first two constants are new to the model, the last two were already present in the first version, but are now given new values. To estimate the model constants, one needs data from subjects in which particular processes stand out clearly. Below, we first describe what data we used to estimate specific constants, and thereafter we describe how the constants were estimated.

To estimate the HL-related model constant $d_{hl,peak}$, which indicates the lipoprotein particle size at which HL activity is highest, we used patients with lipoprotein lipase (LPL) deficiency. In these patients the only remaining lipolysis activity is due to HL. Data on lipoprotein metabolic fluxes in such patients came from Demant, T. et al., supra)

To estimate the model constant $S_{u,liver}$, which helps to model the liver uptake rate at different lipoprotein particle sizes, subjects are needed in which uptake processes take place with least interference from lipolysis processes. By inspecting the kinetic data, we found that normolipidemic ApoE 3/3 subjects met these criteria best. Therefore, $S_{u,liver}$ was estimated using data on lipoprotein metabolic fluxes in ApoE 3/3 subjects from Demant, T. e al., 1991, J. Clin. Invest. 88:1490-1501.

To estimate model constants related to LPL lipolysis, subjects with the ApoE 2/2 genotype were used. Subjects with the ApoE 2/2 isoform are known to have impaired uptake of large VLDL and chylomicron particles. Since VLDL particles can either be taken up by the liver or lipolyzed by LPL, an impaired uptake means that the LPL lipolysis process, that mainly takes place in the VLDL size range, can be distinguished clearly. Also, the lipolysis of smaller particles was found to be impaired in ApoE 2/2 subjects (Demant, T. e al., 1991, J. Clin. Invest. 88:1490-1501), indicating a less effective hepatic lipase function, which should make the LPL activity even more clearly discernable, also for smaller particles. Therefore, the data from subjects with the ApoE 2/2 phenotype were useful for estimating two model constants related to LPL: $\sigma_{a,lpl}$ and $d_{a,lpl\,min}$. Because in apoE 2/2 patients hepatic lipase function is inhibited, the model needed to be adjusted slightly. We allowed the 'HL peak size' ($d_{hl,peak}$) parameter to be optimized for each individual apoE 2/2 subject, which is otherwise constant for all subjects. In this way the model could better handle the special condition of very low HL activity.

In order to determine the model constants via parameter fitting, a double-layered fitting routine was constructed. On the first layer, the algorithm searched for the optimal value for the model constant. The second layer of the routine fitted the parameters of the model at each selected constant value. Both layers used the Levenberg-Marquardt algorithm as implemented in MATLAB's nlinfit method of version 7.7.0 (R2008b) for fitting the constants and parameters respectively. The used error functions can be found in the methods section of the Example. In this way, the model parameters were estimated per individual, while the model constants were estimated for the whole population, using a group of patients judged most suited for the determination of that constant.

We chose to fit the constants in the same order as they were discussed above: first hepatic lipase constants, then liver uptake constants, and finally LPL lipolysis constants. Each time, the newly found constant value was used in the process of fitting the subsequent constants. The order of fitting and the type of subjects, discussed above, were chosen in such a way that the constants that had not yet been fitted exerted minimal influence on the constant being fitted. For instance, the LPL deficient patients used for determining the Hepatic Lipase constants have little LPL activity and little liver uptake activity related to the unknown uptake constants.

The model constants obtained from the optimization process are given in Table 1. The constants show that Hepatic Lipase activity is highest in particles around 31 nm in size, which is the IDL and small VLDL2 size range.

Instead, LPL lipolysis affects particles of approximately 25 nm (lower cutoff) and higher, but the large shape parameter $\sigma_1$ indicates that the LPL rate very gradually increases with size, and really becomes important for the larger VLDL2 and the VLDL1 particles, which LPL is known to affect more. The translation into exact metabolic rates depends on the model parameters that differ for every subject (shown in Table 1).

**Table 1 - Overview of state variables, variables, parameters and constants used in this description**

| State Variables - determine the system state | | |
|---|---|---|
| $d_{i,j}$ | nm | Lipoprotein particle diameter in the i-th step of a lipolysis cascade, in the j-th subclass of the size range covered by that cascade step |
| $Q_{ss}(d_{i,j})$ | Particles * dL⁻¹ | Steady-state particle pool size in a pool with mean particle diameter $d_{i,j}$ |
| Variables - specify processes and output | | |

(continued)

| | | |
|---|---|---|
| $d^{r}_{i,j}$ | nm | the radius of the subclass with average diameter $d_{i,j}$ |
| $J_l(d_{i,j})$ | Particles * dL$^{-1}$ * day$^{-1}$ | Particle flux into the pool with mean particle diameter $d_{i,j}$ due to extrahepatic lipolysis |
| $J_{l,liver}(d_{i,j})$ | Particles * dL$^{-1}$ * day$^{-1}$ | Particle flux into the pool with mean particle diameter $d_{ij}$ due to hepatic lipolysis |
| $k_l(d)$ | day$^{-1}$ | Particle size dependent extrahepatic lipolysis rate |
| $k_{a,liver}(d)$ | day$^{-1}$ | Particle size dependent liver attachment rate (attachment is followed by either lipolysis or uptake) |
| $k_{l,liver}(d)$ | day$^{-1}$ | Particle size dependent liver lipolysis rate |
| $k_{u,liver}(d)$ | day$^{-1}$ | Particle size dependent liver uptake rate |
| $n_{tg}(d_{i,j})$ | Molecules * particle$^{-1}$ | Number of triglyceride molecules in a lipoprotein particle with diameter $d_{i,j}$ |
| $Q^*$ | Particles * dL$^{-1}$ | Steady-state particle pool size in the size range called * |
| $k^*_{u,liver}$ | day$^{-1}$ | Particle size dependent liver uptake rate, averaged per particle over the size range called * |
| $k_i$ | day$^{-1}$ | Particle size dependent extrahepatic lipolysis rate, averaged per particle over all particles in the model |
| $k_{a,liver}$ | day$^{-1}$ | Particle size dependent liver attachment rate, averaged per particle over all particles in the model |
| $J_{p,*}$ | Particles * dL$^{-1}$ * day$^{-1}$ | Particle production flux into the size range called * |
| $J_{in,*}$ | Particles * dL$^{-1}$ * day$^{-1}$ | Particle production influx (production + lipolysis) into the size range called * |
| $Q_{out,([d_a\ d_b])}$ a | Particles * dL$^{-1}$ | Steady state particle pool size in interval from $d_a$ to $d_b$ in the final particle concentration profile. |
| Parameters - are optimized using data | | |
| $k_{l,max}$ | day$^{-1}$ | maximum rate at which extrahepatic lipolysis takes place |
| $k_{a,apoEmax}$ | day$^{-1}$ | maximum rate at which liver binding mediated by ApoE takes place |
| $k_{a,apoB}$ | day$^{-1}$ | rate at which liver binding mediated by ApoB takes place |
| $A$ | nm | shape parameter for liver binding mediated by ApoE |
| $B$ | - | shape parameter for liver binding mediated by ApoE |
| $\sigma_{u,liver}$ | nm | shape parameter describing fraction of liver attachment which is taken up (instead of lipolyzed) - with changing particle size |

(continued)

| Model constants and derived variables - calibrated as described in the Examples | | |
|---|---|---|
| $d_{a,apoEmin}$ | 17 nm | minimum particle diameter at which liver binding mediated by ApoE takes place |
| $d_{hl,peak}$ | 31.33 nm | Hepatic lipase lipolysis peak size (see Eq. 13) |
| $S_{u,liver}$ | 7.87 | Liver uptake shape constant |
| $d_{l,min}$ | 25.13 nm | minimum size at which extrahepatic lipolysis occurs (see Eq. 4 in Schalkwijk et al, 2009) |
| $\sigma_l$ | 77.35 nm | shape constant for extrahepatic lipolysis (see Eq. 4 in Schalkwiik et al, 2009) |

**Cardiovascular and diabetes risk prediction**

When assessing the risk on the occurrence of cardiovascular diseases and metabolic syndrome or diabetes-related diseases, currently such a risk is determined by taking into account various clinical and behavioral parameters ('classical parameters' or 'classical markers').

For cardiovascular diseases such classical parameters are, for instance, the age of the patient, the smoking behavior (either the mere fact of being an (inhaling) smoker or the number of cigarettes per day, the systolic and/or diastolic blood pressure, the HDL cholesterol value (either the concentration or the particle number), the blood glucose concentration, the sex of the subject and the (blood pressure) medication.

These parameters have been assessed in long-term follow up studies, such as the Framingham Heart Study (FHS) (Wilson et al., 1998, Circulation 97:1837-1847; Wang et al., 2003, JAMA 290:1049-1056; D'Agostino et al., 1994, Stroke 25(1):40-43; D'Agostino et al., 2000, Am. Heart J. 139(2 I) 272-281; D'Agostino, 2008, Circulation 118(4):e86), where they have been found to have a predictive characteristic (see also the website dedicated to the FHS (http://www.framin2:hamheartstudy.org/about/index,html). In the FHS the predictive value of these classical parameters on the development of cardiovascular diseases have been investigated in a follow-up study over 10 years in length.

The inventors now have found that the parameters that can be derived from the Particle Profiler analysis are able to improve the predictions made on basis of the 'classical parameters' of risk of cardiovascular diseases. As isshown in the Examples (specifically Example 4) addition of one or more of the parameters in the multivariate risk analysis increases the predictive value for 'general cardiovascular disease'. 'General cardiovascular disease' is a composite endpoint, including coronary death, myocardial infarction, coronary insufficiency, angina, ischemic stroke, hemorrhagic stroke, transient ischemic attack, peripheral artery disease, and heart failure (see http://www.framinghamheartstudy.org/risk/gencardio.html). It has been found that a fair and significant improvement in the result prediction can be achieved when 5 or 6 lipoprotein metabolic ratios derived from the Particle Profiler analysis (which we will refer to in continuation as 'liporatios') are included in the multivariate analysis. The liporatios listed in Table 6 of Example 4 are examples (although optimal) of parameters that can be used for the improvement of the prediction. Addition of further liporatios did not significantly alter the improvement value. Which liporatios to use was determined by calculating for the total set of parameters the improvement caused by adding a single liporatio to the 'classical parameter' set and choosing the one that performed the best. Then, the same was repeated for addition of a second liporatio on the prediction with the set of classical parameters plus the first liporatio. In the same way also the further liporatios were added. However, although the liporatios given in Table 6 of Example 4 are the most optimal predictors, other liporatios sets of two, three, four, five, six or even more liporatios are conceivable that would result in similar effects on the risk analysis. This is mainly due to the fact that there is a large amount of correlation between several of the liporatios that are calculated from the Particle Profiler lipoprotein profile. Thus, while the most optimal parameter for the first addition is the liporatio

$\ln\left(\dfrac{J_{prod,TOT}}{k_{u,liver}^{TOT}}\right)$ this parameter is strongly correlated with any of the following liporatios:

$\ln\left(\dfrac{\overline{k_{u,liver}^{TOT}}}{J_{prod,TOT}}\right)$, $\ln\left(\dfrac{J_{in,ILDL}}{k_{u,liver}^{ILDL}}\right)$,

$$\ln\left(\frac{\overline{k^{ILDL}_{u,\,liver}}}{J_{in,ILDL}}\right), \ \ln\left(\frac{\dfrac{\overline{k^{TOT}_1}}{J_{in,TOT}} + \dfrac{\overline{k^{TOT}_{u,\,liver}}}{J_{in,TOT}}}{2}\right), \ \ln\left(\frac{\dfrac{\overline{k^{TOT}_{lpl}}}{J_{in,TOT}} + \dfrac{\overline{k^{TOT}_{a,\,liver}}}{J_{in,TOT}}}{2}\right), \ \ln\left(\frac{\dfrac{\overline{k^{ILDL}_{lpl}}}{J_{in,ILDL}} + \dfrac{\overline{k^{ILDL}_{u,\,liver}}}{J_{in,ILDL}}}{2}\right).$$

Accordingly, as a first addition to the classical parameters any of the above listed alternatives could have been taken, and the addition thereof would still have a significant impact on the risk analysis. A similar correlation-group can also be established for the second liporatio $\ln\left(\dfrac{J_{in,ILDL}}{k^{ILDL}_{a,\,liver}}\right)$.

The below Table 2 gives the correlation groups for the parameters that are mentioned in Table 6 of Example 4.

Table 2 Correlation groups for Particle Profiler based lipoprotein metabolic ratios (liporatios) for use in risk analysis of cardiovascular disease.

| Addition | Optimal parameter | Highly correlated alternatives |
|---|---|---|
|  |  |  |
| 1 | $\ln\left(\dfrac{J_{prod,TOT}}{k^{TOT}_{u,liver}}\right)$ | $\ln\left(\dfrac{\overline{k^{TOT}_{u,liver}}}{J_{prod,TOT}}\right),\ \ln\left(\dfrac{J_{in,ILDL}}{k^{ILDL}_{u,\,liver}}\right),\ \ln\left(\dfrac{\overline{k^{ILDL}_{u,\,liver}}}{J_{in,ILDL}}\right),$ $\ln\left(\dfrac{\dfrac{\overline{k^{TOT}_1}}{J_{in,TOT}} + \dfrac{\overline{k^{TOT}_{u,liver}}}{J_{in,TOT}}}{2}\right),\ \ln\left(\dfrac{\dfrac{\overline{k^{TOT}_{lpl}}}{J_{in,TOT}} + \dfrac{\overline{k^{TOT}_{a,liver}}}{J_{in,TOT}}}{2}\right),$ $\ln\left(\dfrac{\dfrac{\overline{k^{ILDL}_{lpl}}}{J_{in,ILDL}} + \dfrac{\overline{k^{ILDL}_{u,liver}}}{J_{in,ILDL}}}{2}\right)$ |

(continued)

| Addition | Optimal parameter | Highly correlated alternatives |
|---|---|---|
| 2 | $\ln\left(\dfrac{J_{in,ILDL}}{\overline{k_{a,liver}^{ILDL}}}\right)$ | $\ln\left(\dfrac{J_{in,ILDL}}{\overline{k_{a,liver}^{ILDL}}}\right),\ \ln\left(\dfrac{\dfrac{\overline{k_{hl}^{ILDL}}}{J_{in,ILDL}}+\dfrac{\overline{k_{u,liver}^{ILDL}}}{J_{in,ILDL}}}{2}\right),\ \ln\left(\dfrac{\dfrac{\overline{k_{l}^{ILDL}}}{J_{in,ILDL}}+\dfrac{\overline{k_{u,liver}^{ILDL}}}{J_{in,ILDL}}}{2}\right),$ $\ln\left(\dfrac{\dfrac{\overline{k_{lpl}^{ILDL}}}{J_{in,ILDL}}+\dfrac{\overline{k_{a,liver}^{ILDL}}}{J_{in,ILDL}}}{2}\right),\ \ln\left(\dfrac{\dfrac{\overline{k_{l}^{ILDL}}}{J_{in,ILDL}}+\dfrac{\overline{k_{a,liver}^{ILDL}}}{J_{in,ILDL}}}{2}\right),$ $\ln\left(\dfrac{\dfrac{\overline{k_{l}^{TOT}}}{J_{in,TOT}}+\dfrac{\overline{k_{a,liver}^{TOT}}}{J_{in,TOT}}}{2}\right),\ \ln\left(\dfrac{\dfrac{\overline{k_{hl}^{ILDL}}}{J_{in,ILDL}}+\dfrac{\overline{k_{a,liver}^{ILDL}}}{J_{in,ILDL}}}{2}\right),\ \ln\left(\dfrac{J_{in,TOT}}{\overline{k_{a,liver}^{TOT}}}\right),$ $\ln\left(\dfrac{J_{in,TOT}}{\overline{k_{a,liver}^{TOT}}}\right),\ \ln\left(\dfrac{\dfrac{\overline{k_{hl}^{TOT}}}{J_{in,TOT}}+\dfrac{\overline{k_{u,liver}^{TOT}}}{J_{in,TOT}}}{2}\right)$ |

(continued)

| Addition | Optimal parameter | Highly correlated alternatives |
|---|---|---|
| 3 | $\ln\left(\dfrac{J_{in,ILDL}}{k_1^{ILDL}}\right)$ | $\ln\left(\dfrac{\overline{k_1^{ILDL}}}{J_{in,ILDL}}\right)$, $\ln\left(\dfrac{J_{in,ILDL}}{\overline{k_{hl}^{ILDL}}}\right)$, $\ln\left(\dfrac{\overline{k_{hl}^{ILDL}}}{J_{in,ILDL}}\right)$, $\ln\left(\dfrac{J_{prod,TOT}}{\overline{k_{a,liver}^{TOT}}}\right)$, $\ln\left(\dfrac{\overline{k_{a,liver}^{TOT}}}{J_{prod,TOT}}\right)$, , $\ln\left(\dfrac{\dfrac{\overline{k_1^{ILDL}}}{J_{in,ILDL}}+\dfrac{\overline{k_{a,liver}^{ILDL}}}{J_{in,ILDL}}}{2}\right)$, $\ln\left(\dfrac{\dfrac{\overline{k_{hl}^{TOT}}}{J_{in,TOT}}+\dfrac{\overline{k_{a,liver}^{TOT}}}{J_{in,TOT}}}{2}\right)$, $\ln\left(\dfrac{\dfrac{\overline{k_{hl}^{TOT}}}{J_{prod,TOT}}+\dfrac{\overline{k_{a,liver}^{TOT}}}{J_{prod,TOT}}}{2}\right)$, $\ln\left(\dfrac{\dfrac{\overline{k_{hl}^{TOT}}}{J_{prod,TOT}}+\dfrac{\overline{k_{u,liver}^{TOT}}}{J_{prod,TOT}}}{2}\right)$ |
| 4 | $\ln\left(\dfrac{\overline{k_1^{ILDL}}}{k_{u,liver}^{ILDL}}\right)$ | $\ln\left(\dfrac{\overline{k_{u,liver}^{ILDL}}}{k_1^{ILDL}}\right)$, $\ln\left(\dfrac{\overline{k_1^{ILDL}}}{k_{a,liver}^{ILDL}}\right)$, $\ln\left(\dfrac{\overline{k_{u,liver}^{ILDL}}}{k_{hl}^{ILDL}}\right)$, $\ln\left(\dfrac{\overline{k_{a,liver}^{ILDL}}}{k_{hl}^{ILDL}}\right)$, |

(continued)

| Addition | Optimal parameter | Highly correlated alternatives |
|---|---|---|
| | | $\ln\left(\dfrac{\overline{k_{hl}^{ILDL}}}{k_{u,liver}^{ILDL}}\right)$, $\ln\left(\dfrac{\overline{k_{a,liver}^{ILDL}}}{k_{u,liver}^{ILDL}}\right)$, $\ln\left(\dfrac{\overline{k_{hl}^{ILDL}}}{k_{a,liver}^{ILDL}}\right)$, $\ln\left(\dfrac{\overline{k_{u,liver}^{ILDL}}}{k_{a,liver}^{ILDL}}\right)$, $\ln\left(\dfrac{\overline{k_{u,liver}^{TOT}}}{k_{hl}^{TOT}}\right)$, $\ln\left(\dfrac{\overline{k_{a,liver}^{TOT}}}{k_{hl}^{TOT}}\right)$, $\ln\left(\dfrac{\overline{k_{hl}^{TOT}}}{k_{u,liver}^{TOT}}\right)$, $\ln\left(\dfrac{\overline{k_{u,liver}^{TOT}}}{k_{a,liver}^{TOT}}\right)$, $\ln\left(\dfrac{\overline{J_{in,TOT}}}{k_{hl}^{TOT}}\right)$, $\ln\left(\dfrac{\overline{k_{hl}^{TOT}}}{J_{in,TOT}}\right)$ |
| 5 | $\ln\left(\dfrac{\overline{k_{a,liver}^{ILDL}}}{k_{l}^{ILDL}}\right)$ | (see 4) |
| 6 | $\ln\left(\dfrac{\overline{k_{hl}^{ILDL}}}{k_{l}^{ILDL}}\right)$ | $\ln\left(\dfrac{\overline{k_{lpl}^{ILDL}}}{k_{hl}^{ILDL}}\right)$, $\ln\left(\dfrac{\overline{k_{l}^{ILDL}}}{k_{hl}^{ILDL}}\right)$, $\ln\left(\dfrac{\overline{k_{lpl}^{ILDL}}}{k_{l}^{ILDL}}\right)$, $\ln\left(\dfrac{\overline{k_{lpl}^{ILDL}}}{k_{a,liver}^{ILDL}}\right)$, $\ln\left(\dfrac{\overline{k_{lpl}^{ILDL}}}{J_{in,ILDL}}\right)$, $\ln\left(\dfrac{\overline{k_{lpl}^{ILDL}}}{k_{u,liver}^{ILDL}}\right)$ |

According to a preferred embodiment of the invention, in the method of improving the risk analysis for determining the risk of developing a cardiovascular disease, the additional liporatios(s) that can be added to the multivariate analysis is(are) selected from group 1 of the above table 2, more preferable one from group 1 and one from group 2, more preferable one from group 1, one from group 2 and one from group 3, more preferable one from group 1, one from group 2, one from group 3 and one from group 4, more preferable one from group 1, one from group 2, one from group 3, one from group 4 and one from group 5, and even more preferable one from each of the groups 1-6. It is to be understood that 'group' as mentioned with respect to the above Table means both the optimal liporatio and the liporatios that are highly correlated therewith. Most preferable the liporatios that are combined with the classical parameters are the parameters as provided in Table 6 of Example 4 (i.e. the optimal parameters from the above Table 2).

Use of the additional liporatios derived from the lipoprotein profile in risk analysis, yields a more reliable prediction of the risk on cardiovascular diseases. Such a cardiovascular disease may be a disease selected from the group of atrial fibrillation, (congestive) heart failure, coronary heart disease, intermittent claudication, stenosis, thromboembolism, myocardial infarction, coronary insufficiency, angina, ischemic stroke, hemorrhagic stroke, ischemia, and peripheral artery disease.

Accordingly, on basis of a multivariate analysis as shown in the Examples, a more reliable risk determination of the occurrence of a cardiovascular disease in a subject in the near future can be given. Of course, an improved risk calculation is of importance for determining the treatment or diet of such a subject.

In another embodiment of the invention, lipoprotein metabolic ratios from the lipoprotein profile such as calculated by the Particle Profiler system ('liporatios') may also be advantageously used to improve the prediction for diseases related to the metabolic syndrome, such as diabetes, especially diabetes type 2, obesitas, insulin resistance, or any other pre-diabetic condition (see Example 5)

In this case the 'classical parameters' differ slightly from the classical parameter set as developed for cardiovascular diseases. The classical set for (pre-)diabetic conditions comprises: the glucose level in the blood, the body mass index (BMI), smoking behaviour of the spouse of the subject, the sex of the subject, the HDL cholesterol value and the diastolic blood pressure. This set may be completed with the HDL and VLDL particle number.

Now, as shown in Example 5, addition of one, two, three or more liporatios from the Particle profiler system significantly improves the general diabetes risk prediction from a multivariate analysis based on the classical parameters.

Again, as discussed above for cardiovascular disease risk prediction the parameters as given in Table 9 of Example 5 are optimal parameters, but they can be replaced by highly correlated alternatives as given in the below Table 3.

Table 3. Correlation groups for Particle Profiler based lipoprotein metabolic ratios (liporatios) for use in risk analysis of diseases related to metabolic syndrome.

| Addition | Optimal parameter | Highly correlated alternatives |
|---|---|---|
| | | |
| 1 | $\ln\left(\dfrac{J_{in,ILDL}}{k_{u,liver}^{ILDL}}\right)$ | $\ln\left(\dfrac{\overline{k_{u,liver}^{ILDL}}}{\overline{J_{in,ILDL}}}\right)$, $\ln\left(\dfrac{J_{in,TOT}}{\overline{k_{u,liver}^{TOT}}}\right)$, $\ln\left(\dfrac{\overline{k_{u,liver}^{TOT}}}{\overline{J_{in,TOT}}}\right)$, $\ln\left(\dfrac{J_{prod,TOT}}{\overline{k_{u,liver}^{TOT}}}\right)$, $\ln\left(\dfrac{\overline{k_{u,liver}^{TOT}}}{J_{prod,TOT}}\right)$, $\ln\left(\dfrac{\dfrac{\overline{k_{lpl}^{TOT}}}{J_{in,TOT}}+\dfrac{\overline{k_{u,liver}^{TOT}}}{J_{in,TOT}}}{2}\right)$, $\ln\left(\dfrac{\dfrac{\overline{k_{lpl}^{ILDL}}}{J_{in,ILDL}}+\dfrac{\overline{k_{u,liver}^{ILDL}}}{J_{in,ILDL}}}{2}\right)$ |
| 2 | $\ln\left(\dfrac{\overline{k_{lpl}^{ILDL}}}{\overline{k_{a,liver}^{ILDL}}}\right)$ | $\ln\left(\dfrac{\overline{k_{lpl}^{ILDL}}}{\overline{k_{u,liver}^{ILDL}}}\right)$, $\ln\left(\dfrac{\overline{k_{lpl}^{ILDL}}}{J_{in,ILDL}}\right)$, $\ln\left(\dfrac{\overline{k_{lpl}^{ILDL}}}{\overline{k_{hl}^{ILDL}}}\right)$, $\ln\left(\dfrac{\overline{k_{l}^{ILDL}}}{\overline{k_{hl}^{ILDL}}}\right)$, $\ln\left(\dfrac{\overline{k_{hl}^{ILDL}}}{\overline{k_{l}^{ILDL}}}\right)$, $\ln\left(\dfrac{\overline{k_{lpl}^{ILDL}}}{\overline{k_{l}^{ILDL}}}\right)$ |
| 3 | $\ln\left(\dfrac{\overline{k_{hl}^{ILDL}}}{\overline{k_{l}^{ILDL}}}\right)$ | $\ln\left(\dfrac{\overline{k_{lpl}^{ILDL}}}{\overline{k_{hl}^{ILDL}}}\right)$, $\ln\left(\dfrac{\overline{k_{l}^{ILDL}}}{\overline{k_{hl}^{ILDL}}}\right)$, $\ln\left(\dfrac{\overline{k_{lpl}^{ILDL}}}{\overline{k_{l}^{ILDL}}}\right)$, $\ln\left(\dfrac{\overline{k_{lpl}^{ILDL}}}{\overline{k_{a,liver}^{ILDL}}}\right)$, $\ln\left(\dfrac{\overline{k_{lpl}^{ILDL}}}{J_{in,ILDL}}\right)$, $\ln\left(\dfrac{\overline{k_{lpl}^{ILDL}}}{\overline{k_{u,liver}^{ILDL}}}\right)$ |

According to a preferred embodiment of the invention, in the method of improving the risk analysis for determining the risk of developing a diabetic condition, the additional liporatios(s) that can be added to the multivariate analysis is (are) selected from group 1 of the above table 2, more preferable one from group 1 and one from group 2, more preferable one from group 1, one from group 2 and one from group 3. Most preferable the liporatios that are combined with the classical parameters are the parameters as provided in Table 9 of Example 5 (i.e. the optimal parameters from the above Table 3)

Use of the additional parameter(s) of the lipoprotein profile in risk analysis yields a more reliable prediction of the risk on (pre-)diabetic conditions. Such a (pre-)diabetic condition may be a disease selected from the group of diabetes, in particular diabetes type 2, insulin resistance, impaired glucose tolerance, metabolic syndrome, and obesitas. It is thus clearly shown in the present invention that parameters (liporatios) that are derived from the Particle profiler system can be applied for improving the risk analysis for cardiovascular diseases and for (pre-)diabetic conditions.

**Examples**

**Model Parametrisation: Overview**

The model equations have been parameterized as follows: **Production:** no fitted parameters (eq. 1&2 in van Schalkwijk et al. 2009, *supra)*

The $J_{in}$'s are based directly on the dataset being fitted. Extrahepatic lipolysis: 1 fitted parameter (eq. 4 in van Schalkwijk et al. 2009, *supra*.) $k_{l,max}$ maximum rate at which extrahepatic lipolysis takes place 2 fixed model constants: $d_{l,min}$ minimum size at which extrahepatic lipolysis occurs $\sigma_l$ shape parameter for extrahepatic lipolysis

**Liver attachment, lipolysis and uptake:** 5 fitted parameters (eq. 6&10)

$k_{a,apoEmax}$ maximum rate at which liver binding mediated by apoE takes place

$k_{a,apoB}$ rate at which liver binding mediated by apoB takes place *A* shape constant for liver binding mediated by apoE *B* shape parameter for liver binding mediated by apoE

$\sigma_{u,liver}$ shape parameter describing how the fraction of liver attachment which is taken up (instead of lipolyzed) varies with particle size.

2 fixed model constants

$S_{u,liver}$ shape constant describing how the fraction of liver attachment which is taken up (instead of lipolized) varies with particle size.

$d_{a,apoEmin}$ minimum particle diameter at which liver binding mediated by apoE takes place

**Triglyceride loss during lipolysis** (eq 8 in van Schalkwijk et al., 2009, *supra)*

1 fixed model constant

$f_{ig}$ fraction of triglycerides lost at each lipolysis step

**Model reparameterisation for fitting of flux data**

Using the parameters specified above, the fitting routine had difficulty to find the global minimum mean square error. In order to improve its performance we specified a reparametrization, specific for the data type used herein. This allows the fitting routine to find a minimum without difficulty.

$k_{l,max}$ maximum rate at which extrahepatic lipolysis takes place

(unchanged from original model)

*B* shape parameter for liver binding mediated by apoE.

(unchanged from original model)

$\sigma_{u,liver}$ shape parameter describing how the fraction of liver attachment which is taken up (instead of lipolized) varies with particle size.

(unchanged from original model)

$k_{a,apoB}$ the liver attachment rate due to apoB-related processes (unchanged from original model)

$k_{peak}$ the total rate of all processes at the particle size at which hepatic lipolysis is at its peak ($d=d_{hl,peak}$).

Fixed model constant

$d_{hl,peak}$ Size at which hepatic lipolysis is at its peak The new parameter is specified as follows:

$$k_{peak} = k_{a,liver}(d_{hl,peak}) + k_l(d_{hl,peak}) \qquad eq.\ 12$$

This specification of parameters may lead to problems in the fitting routine in specific cases, when the boundary values of processes are reached. Therefore, during the parameter conversion process from fitted parameters to parameters for model calculation, the following fitted parameters were dynamically set to their boundary value. This procedure means that a parameter is only set to its boundary value if necessary, otherwise it is fitted normally.

$$\text{Condition:}\quad k_{\text{peak}} \le k_{a,\text{apoB}} + k_{l,\text{max}}$$

Set boundary: $k_{\text{peak}} = k_{\text{a,apoB}} + k_{l,\max} + 0.00001$

Condition: $\sigma_{u,liver} > 25$

Set boundary: $\sigma_{u,liver} = 25$

Condition: $B < 1 + e^{\ln\left(\frac{d - d_{a,liver\_\min}}{s_{u,liver}^{1/2} \cdot \sigma_{u,liver}}\right) s_{u,liver}} \cdot s_{u,liver}$

Set boundary: $B = 1 + e^{\ln\left(\frac{d - d_{a,liver\_\min}}{s_{u,liver}^{1/2} \sigma_{u,liver}}\right) s_{u,liver}} \cdot s_{u,liver} + 0.000001$

**Size classes**

The size range of each size class has been estimated as follows, modified from [31]:

Table 4. Size ranges of classes of lipoproteins

| Subfraction | Minimum size | Maximum size |
|---|---|---|
| LDL | 5 | 25.0 |
| IDL | 25.0 | 30.0 |
| VLDL2 | 30.0 | 36.0 |
| VLDL1 | 36.0 | 60 |

**Error function**

The nlinfit routine calculates a sum of square difference between data points and model predictions. Before entering into this routine the data was scaled a) to correct for different units of pools data and flux data and b) to indicate the relative importance of each data point. This adjustment is specific for the type of data used. Data and model predictions were divided by the following scaling factors:

Table 5. Scaling factors for Particle profiler model.

| | LDL | IDL | VLDL 2 | VLDL 1 |
|---|---|---|---|---|
| Particle pool scale factor (particles / fl) | 3 | 2 | 2 | 1 |
| Lipolysis efflux scale factor (min$^{-1}$) | - | 0.005 | 0.005 | 0.005 |
| Uptake flux scale factor (min$^{-1}$) | 0.005 | 0.005 | 0.005 | 0.005 |

The lower weights of the larger particle pools give them more importance in the fitting routine. This is desirable since these pools are also important to estimate the rate of the fluxes correctly.

To indicate the error value between the data and the model fit a percentage error was defined. This score was not used for model fitting, only for reporting an intuitive error score. It takes into account the number of data points for pools, uptake and lipolysis and the higher importance (double of the flux total) of the pool sizes; they are most important for parameter estimation. It is given by the following formula:

$$E = \frac{16}{23} \cdot \frac{\sum_i \left| Q_i^d - Q_i^m \right|}{\sum_i Q_i^d} + \frac{3}{23} \frac{\sum_i \left| J_i^{l,d} - J_i^{l,m} \right|}{\sum_i J_i^{l,d}} + \frac{4}{23} \frac{\sum_i \left| J_i^{u,d} - J_i^{u,m} \right|}{\sum_i J_i^{u,d}}$$

*eq. 13*

where E stands for error value, Q indicates the pool size, superscript d indicating the data and *m* the model fit; *J* stands for a flux, superscripts d and m as before, *l* indicates lipolysis, *u* indicates uptake. Subscript *i* indexes the different data points of each lipolysis size class, i.e. LDL, IDL, VLDL2 and VLDL1.

**Conversions**

The datasets of Packard et al.(2000, J. Lipid Res. 41 :305-318), Demant et al. (1993, J. Lipid Res. 34 :147-156 ; 1991, J. Clin. Invest. 88 :1490-1501), and Bilz et al.(2004, J. Lipid Res. 45 :174-185) contain estimations for the lipoprotein pools of the various classes in mg, and turnover speeds in pools per day. These are converted to particle concentrations and particle fluxes respectively. This needs the assumption that only ApoB-100 is present on lipoprotein particles in the fasted state, which is reasonable given that apoB-48 is produced by the intestine mainly postprandially.

$$n\left(\frac{mol}{L}\right) = \frac{n(g)}{M_{ApoB-100}(g/mol) \cdot V_{blood}(L)}$$

*eq. 14*

where n is the number of lipoproteins, $M_{ApoB-100}$ the molar mass of ApoB-100 and $V_{blood}$ the blood volume of an individual person (taken to be 5 L).

**Example 1**

Comparison of new model with model described in van Schalkwijk et al, 2009 and WO 2009/151324.

With the new model equation and settings, a first test examined whether the results of the model validation in our first paper still hold. For this test, the subjects reported by Packard et al. (Packard, C.J. et al., 2000, J. Lipid Res. 41:305-318) were fitted with the improved model and the results the results were compared with those from the first model implementation. In the first implementation, the model reproduced a shift in 'LDL peak size', which was independently measured. The model analysis also identified credible changes in relevant biological processes between groups with differing 'LDL peak size'. For the new model, we judged how well the data was fitted, whether the shift in 'LDL peak size' was still reproduced by the model, and whether the model still identified similar differences in biological processes between groups with differing 'LDL peak size'.

The model with optimized constants and one free parameter less than in the first implementation, reproduced the data from Packard et al. well. The overall fit error, defined in Schalkwijk et al., 2009, was 7.3% $\pm$ 3.6%, compared to a 7.2% $\pm$ 4.5% error in our first paper. In Schalkwijk et al., 2009, the model calculated a difference in LDL size of 4.2 nm between subjects with phenotype 'A' (large LDL particles) versus phenoty-pe B' (small LDL particles). This LDL size difference was significant, with a Kruskal-Wallis p-value of 0.026. In the new implementation we saw a similar difference, although with 1.9 nm it was less pronounced. The Kruskal-Wallis test indicated a trend, with p=0.089. The most likely cause of this difference is the description of hepatic lipolysis, which lost one free parameter in the new model. This conjecture was confirmed by studying the difference in metabolic processes between subjects with phenotypes 'A' and 'B' from Packard et al., as analyzed by our initial model versus the new implementation. The latter are shown in table 2. As observed earlier (Schalkwijk et al., 2009), we saw differences in the average particle lipolysis rate in VLDL 1 and VLDL 2, and in the average particle uptake rate in LDL between 'A' and 'B' phenotypes. In contrast to our first study, no differences between the two phenotypes were found for HL lipolysis in LDL. Instead differences were found in uptake of IDL particles and LPL lipolysis of VLDL and IDL. For particle age, similar differences between 'A' and 'B' phenotypes were found in LDL, VLDL2 and VLDL1, as well as an additional difference in IDL age. For particle size, differences between phenotypes were found for LDL and IDL, and for the new implementation an additional difference in VLDL1 particle diameter was found. Overall this comparison indicates that the new implementation made the model less sensitive to changes in LDL metabolism, but increased its power to identify changes in LPL lipolysis in the VLDL and IDL range.

**Example 2**

Model corroboration

Particle Profiler calculates metabolic process rates averaged per particle, and distinguishes between HL and LPL lipolysis. Because these quantities cannot be measured directly, the best way of validating the model is through corroboration. Patients with different metabolic conditions need to be modeled equally well. Therefore, we applied Particle Profiler to lipoprotein kinetics data from the following studies:

Demant, T. et al., 1993, J. Lipid Res. 34:147-156
Demand, T. et al., 1991, J. Clin. Invest. 88:1490-1501
Packard, C. et al., 2000, J. Lipid Res. 41:305-318
James, R. et al., 1989, J. Lipid Res. 30:159-169
Demand, T. et al., 1996, Am. J. Physiol. 270:E 1022-E 1036
Demand, T. Et al., 1998, Kidney Int. 54:2064-2080
Lundahl, B. et al., 2006, Am. J. Physiol. Endocrinol. Metab. 290:E739-E745
Schmitz, M. et al., 2001, J. Acquir. Immune Defic. Syndr. 26:225-235
Bilz, S. et al., 2004, J. Lipids Res. 45:174-185
Forster, L. et al., 2002, Atherosclerosis 164:129-145
Packard, C. et al., 1993, J. Clin. Endocrinol. Metab. 76:1209-1216

In these studies the lipid metabolism data of subjects with a wide range of dyslipidemias that the model had not yet analyzed before were provided. We inspected whether the model was able to fit data from all of these studies well. If successful, this indicates that the biology incorporated in the model in the form of mathematical equations is adequate to describe the measured experimental data, or in other words that the model is consistent with reality.

The overall fit error for the data from the different lipoprotein kinetics studies was 10.4% with a standard deviation of 6.5%. The highest errors were found when analyzing data from Demant et al. 1998, where the fit error was 20.1%±6.4%. Many subjects in this study, including the controls, had a high VLDL2 pool that our model was unable to reproduce in conjunction with the reported flux values. Without these subjects, the average fit error was 8.8%±5.0%.

**Example 3**

Lipoprotein metabolic ratios for VLDL metabolism

Since overproduction of large VLDL particles is an important characteristic of the atherogenic lipoprotein phenotype (Austin, M. et al., 1990, Circulation 82:495-506), we first examined VLDL metabolism. The only current clinical marker reflecting VLDL is total plasma triglycerides, but this marker also includes triglycerides in chylomicrons, IDL, LDL and HDL. The marker introduced here relates more specifically to VLDL.

Based on Particle Profiler, we derived ratios between hepatic VLDL uptake and production, and between LPL-related VLDL lipolysis and production. Since the model calculates the metabolic rates of VLDL at each particle size, this complex information needs to be integrated into a single value. Therefore, we calculated three ratios of metabolic rates of a VLDL particle. These are

$$\frac{\overline{k_{u,liver}^{VLDL}}}{J_{p,VLDL}}, \quad \frac{\overline{k_{l}^{VLDL}}}{J_{p,VLDL}},$$

and VLDL performance, whose mathematical definition can be found below. A schematic introduction can be found in Figure 12. When referring to the 'VLDL metabolism ratios', we refer to these three ratios.

The VLDL uptake - production ratio

$$\frac{\overline{k_{u,liver}^{VLDL}}}{J_{p,VLDL}}$$

can be calculated from modeled
values as follows:

$$\frac{\overline{k_{u,liver}^{VLDL}}}{J_{p,VLDL}} = \frac{R + \sum_{d_{i,j} \geq d_a + \frac{1}{2}d_{i,j}^r}^{d_{i,j} \leq d_b - \frac{1}{2}d_{i,j}^r} k_{u,liver}(d_{i,j}) \cdot \frac{Q_{ss}(d_{i,j})}{Q_{VLDL}}}{J_{p,VLDL}} \qquad eq.\ 15$$

where $k_{u,liver}(d_{i,j})$ is the hepatic uptake rate, and $Q_{ss}(d_{i,j})$ is the concentration of particles in the subclass with average diameter $d_{i,j}$. The meaning of subindices i and j relates to the position of the subclass in the lipolysis cascade, which is explained in van Schalkwijk et al. 2009, under 'lipolysis cascades'. $Q_{VLDL}$ and $J_{p,VLDL}$ are the total concentration of VLDL particles and total VLDL production influx respectively, where VLDL covers the size range from $da$=30 nm to $d_b$=80 *nm*. R is the linearly interpolated small remainder for the boundary subclasses, which partially fall in the selected range:

$$R_{low} = \frac{\left(d_{i,j} + \frac{1}{2}d_{i,j}^r\right) - d_a}{d_{i,j}^r} \cdot k_{u,liver}(d_{i,j}) \cdot \frac{Q_{ss}(d_{i,j})}{Q_{out}([d_a, d_b])} \quad where \quad d_a \in [d_{i,j} - \frac{1}{2}d_{i,j}^r, d_{i,j} + \frac{1}{2}d_{i,j}^r]$$

$$R_{high} = \frac{d_b - \left(d_{i,j} - \frac{1}{2}d_{i,j}^r\right)}{d_{i,j}^r} \cdot k_{u,liver}(d_{i,j}) \cdot \frac{Q_{ss}(d_{i,j})}{Q_{out}([d_a, d_b])} \quad where \quad d_b \in [d_{i,j} - \frac{1}{2}d_{i,j}^r, d_{i,j} + \frac{1}{2}d_{i,j}^r]$$

$$R = R_{high} + R_{low}$$

(eq.16).

wherein $d_{i,j}^r$ represents the radius of the subclass with average diameter $d_{i,j}$.

The ratio between LPL-related lipolysis and production in VLDL

$$\left(\frac{\overline{k_l^{VLDL}}}{J_{p,VLDL}}\right)$$

is
defined analogously, by replacing $k_{u,liver}(d_{i,j})$ in equations 4 and 4a by $k_l(d_{i,j})$. To show in a single value how efficiently produced VLDL particles are metabolized, we introduce the 'VLDL performance' diagnostic, which is the average of the previous two ratios.

*VLDLperformance*

$$= \frac{\dfrac{\overline{k_{u,liver}^{VLDL}}}{J_{p,VLDL}} + \dfrac{\overline{k_l^{VLDL}}}{J_{p,VLDL}}}{2}$$

Both ratios and VLDL performance have the dimension *volume * particles^{-1}*.
Figure 12 graphically shows how VLDL performance and the two ratios are related, and how different disturbances in VLDL metabolism affect VLDL performance.

**General dyslipidemia**

In order to obtain an indication of the clinical relevance of our new markers for VLDL metabolism, we applied the Particle Profiler model to a range of published studies with pooled lipoprotein flux data obtained in dyslipidemic subjects. In nearly all these studies, a group of healthy subjects and a group of patients showing a specific type of dyslipidemia were investigated and compared. Our pool of dyslipidemic subjects was defined as all the subjects that were considered to be 'dyslipidemic' in each study. To be included in our analysis, the studies also had to report data of standard clinical chemistry for comparison with our new markers. The set of healthy subjects included normolipidemic control subjects from studies by James *et al. (supra)* and Demant *et al.* (1996 and 1998, *supra),* apoE 3/3 subjects from Demant *et al.* 1991 (*supra*), phenotype 'A' (large LDL particle size) subjects from Packard et al. (2000, *supra)* as well as all subjects from Lundahl *et al. (supra).* The set of dyslipidemic subjects included two subject groups showing mixed dyslipidemia prior to treatment by Bilz *et al. (supra)* and Forster *et al. (supra),* kidney patients from Demant *et al.* (1998, *supra),* hypothyroid subjects with and without T4 therapy from Packard *et al.* (1993, *supra),* HIV treatment-associated hyperlipidemic subjects from Schmitz *et al. (supra)* and phenotype 'B' subjects, with small LDL particle size, from Packard *et al.* 2000. The latter study was also included in our first paper (van Schalkwijk et al, 2009).

We tested whether Particle Profiler-derived VLDL performance was different for normolipidemic versus dyslipidemic subjects. This test also examined the difference in VLDL performance in relation to differences in triglycerides, HDL cholesterol and LDL cholesterol. These differences were expressed as the ability to correctly predict the known normolipidemic or dyslipidemic state from the diagnostic parameters. The test consisted of two phases: first the predictive power of each diagnostic separately, then the predictive power of multivariate models. For the multivariate models we performed logistic regression, subsequently adding LDL cholesterol, HDL cholesterol, triglycerides and VLDL performance as predictor variables. The diagnostic accuracy, was quantified using ROC curves (Obuchowski, N., 2005, AJR Am. J. Roentgenol. 184:364-372). We used both the Area Under the Curve (AUC) and Partial Area Under the Curve statistics (pAUC - for false positive rates < 0.2) to quantify the predictive power of each separate diagnostic, and of each multivariate regression model.

The new VLDL performance marker clearly differed between normolipidemic and dyslipidemic subjects. Figure 13a shows ROC curves that describe how well LDL cholesterol, HDL cholesterol, triglycerides, and VLDL performance distinguished normolipidemic from dyslipidemic subjects. Figure 13b shows ROC curves of multivariate regression models that successively incorporate these diagnostics for making the same distinction together. Table 3 shows the partial area-under-the-curve (pAUC) and area-under-the-curve AUC values, indicating diagnostic power for dyslipidemia, for each of the regression models. The table shows that VLDL performance distinguished normolipidemics better from dyslipidemics than the routine clinical chemistry parameters. Also, VLDL performance improved the distinction when used in combination with LDL cholesterol, HDL cholesterol and triglycerides. When accepting no false positives, the model including VLDL performance had a 91% sensitivity for correctly identifying dyslipidemic subjects, versus a 67% sensitivity when using only triglycerides, HDLc and LDLc. Therefore, VLDL performance had additional value for distinguishing dyslipidemic from normolipidemic subjects compared to standard diagnostics.

**Dyslipidemic subgroups**

Next, we examined the average value of our VLDL-related diagnostic parameters for each of the studied subgroups. This comparison included subject groups for which no standard clinical chemistry parameters were available (normolipidemic subjects from [16-21]), and subject groups with specific genetic disorders. These genetic disorders include LPL -/- [15], apoE 2/2 [16], apoE 4/4 [16], homozygous familial hypercholesterolemia [18], homozygous familial defective apoB [28], and S447X [29], a single nucleotide polymorphism in the LPL gene.

Figure 14 shows the average VLDL metabolism-ratios for all included subject groups. The figure clearly indicates that the normolipidemic groups (green and light-green lines) had a higher VLDL performance (projection onto the identity line) than dyslipidemic groups. Interestingly, this mainly seems to be due to an increased LPL lipolysis - production ratio in VLDL, although the liver uptake - production ratio in VLDL is generally also higher. In the dyslipidemic patients, the VLDL ratios were lower to a different extent. Hypothyroid patients on T4 treatment (marked with '1') seemed to have an improved VLDL performance with respect to the untreated patients (marked with '4'). Patients with small LDL particles showed a relatively light dyslipidemia, whereas mixed hyperlipidemia was associated with different degrees of dyslipidemia ('3' and '8'). Kidney patients, hypothyroid patients and HIV-treated patients fell in between the mixed hyperlipidemias. The patients with genetic deficiencies showed up at the expected locations. LPL deficient patients had the lowest VLDL performance, and also the S447X polymorphism in this gene negatively effected VLDL performance. ApoE 4/4 subjects that are known to display a good VLDL clearance, showed up together with the normolipidemics, whereas apoE 2/2 subjects, known to have impaired VLDL clearance, showed up as slightly dyslipidemic.

**Response to treatment**

A diagnostic's clinical usefulness increases if there are treatment options when the diagnostic indicates illness. Therefore, we investigated how our VLDL-related diagnostic parameters respond to treatment. For this purpose we used data on atorvastatin and fenofibrate treatment in mixed dyslipidemic subjects from Bilz *et al.* , and atorvastatin and simvastatin treatment in mixed dyslipidemic subjects from Forster et al.. All these studies contain lipoprotein kinetics data at baseline and after each treatment, which we used as input for Particle Profiler.

Figure 15 shows how the VLDL metabolism diagnostics responded to treatment in mixed dyslipidemic patients. Fenofibrate and simvastatin clearly raised VLDL performance values. The effect of atorvastatin was borderline significant in the study by Forster *et al.* , while the study by Bilz *et al*. had too few subjects to distinguish this effect. Therefore, we conclude that fenofibrate and simvastatin have a stronger effect on VLDL performance than atorvastatin.

**Example 4**

Lipoprotein metabolic ratios improve cardiovascular risk prediction.

The Framingham Risk score predicts cardiovascular risk based on six varables: age, diabetes, smoking, treated and untreated systolic blood pressure, total cholesterol, and HDL cholesterol. Newer lipoprotein measurement methods have attempted to improve risk prediction by quantifying lipoprotein subclasses by size [NMR, HPLC, AF4, ion mobility] or density [VAP] range. The diagnostics that are most often derived from these data are LDL and HDL particle number , although other multivariate measures have been proposed.

Recently, we have developed a computational model to analyze measured lipoprotein subclass profiles in terms of the underlying metabolic activity. The model can calculate ratios of lipoprotein metabolic processes from a single lipoprotein profile measurement. Since metabolic disorders are at the basis of cardiovascular disease, we hypothesized that lipoprotein metabolism ratios can improve cardiovascular disease prediction. We evaluated this hypothesis for subjects from the Framingham offspring cohort.

**METHODS**

**Study Sample and Risk Factors**

In this study we used subjects from the Offspring cohort in the Framingham Heart Study (FHS) (see www.framinghamheartstudy.org). Subjects were included when they had no history of cardiovascular disease, gave written informed consent for general research use, had complete NMR lipoprotein profiles recorded, and had a complete record of classical cardiovascular risk factors.

**Computational Modeling**

We applied the Particle Profiler computational model to the NMR lipoprotein profiles. All lipoprotein metabolic ratios can be expressed by the following general expressions:

$$\ln\left(\frac{\overline{k^{range}_{rateprocess}}}{J_{fluxprocess,range}}\right) \qquad \ln\left(\frac{J_{fluxprocess,range}}{\overline{k^{range}_{rateprocess}}}\right)$$

$$\ln\left(\frac{\overline{k^{range}_{rateprocess\,1}}}{\overline{k^{range}_{rateprocess\,2}}}\right)$$

wherein

$$\overline{k^{range}_{rateprocess}}$$

indicates the particle size dependent rate of the
process denoted by *rateprocess,* averaged per particle over the size range denoted by *range,* wherein *rateprocess* is one of the following processes: *lpl* (LPL-related lipolysis), *hl* (HL-related lipolysis), 1 (total lipolysis, sum of LPL- and HL-related lipolysis), *u,liver* (liver uptake), or α, *liver* (liver attachment) and wherein *range* is one of the following size ranges: ILDL (IDL and LDL size range, 5-30 nm in the model), VLDL (VLDL size range, 30-80 nm in the model) or TOT (complete modeled size range, 5-80 nm in the model). Please note that the model does not include HDL, so these particles are not included in the smaller size range.

$J_{\text{fluxprocess,range}}$ indicates the particle flux denoted by *fluxprocess* into the size range denoted by *range*, wherein *fluxprocess* is one of the following processes: *prod* (direct production from the liver) or *in* (total influx due to both production and lipolysis of larger particles) and wherein *range* is one of the size ranges mentioned above.

The input dataset for constructing the multivariate prediction model consisted of all ratios indicated by expressions 1, 2, and 3. Because of the large
number of zero entries, ratios with

$$\overline{k_{\text{lpl}}^{\,range}}$$

or $J_{prod,ILDL}$ in the denominator
were excluded.

In addition, several averages of ratios were constructed, of the form:

$$\ln\left(\frac{\dfrac{\overline{k_{\text{rateproces sA}}^{\,range}}}{J_{\text{fluxproces s,range}}} + \dfrac{\overline{k_{\text{rateproces sB}}^{\,range}}}{J_{\text{fluxproces s,range}}}}{2}\right)$$

wherein rateprocessA is one of the following processes: *lpl, hl,* or l; rateprocessB is either *a,liver* or *u, liver.* All other symbols are defined as described above. Ratios including $J_{prod,ILDL}$ were excluded, because of the large number of zero entries.

These expressions resulted in 124 lipoprotein metabolic ratios from expressions 1, 2, and 3 and 30 from expression 4, which totalled 154 lipoprotein metabolic ratios.. We calculated ratios of all modeled processes (lipoprotein production, total lipoprotein lipolysis, HL lipolysis, LPL lipolysis, liver lipoprotein attachment, liver lipoprotein uptake) in each of three sets of lipoprotein size ranges (VLDL through LDL, VLDL only, IDL through LDL).

**Outcomes**

Subjects who experienced a cardiovascular event, as defined by the Framingham study, up to 10 years after the NMR measurement, were designated as 'cases', others as 'controls'.

**Statistical Analysis**

We used multivariate logistic regression to correlate predictor variables with the CVD outcome. We grouped predictor variables into three sets: 1. classical cardiovascular risk parameters, 2. direct lipoprotein profile-based parameters, and 3. Particle Profiler-based lipoprotein metabolic ratios (liporatios); a complete overview of the variables in these sets can be found in the below table 6:

We constructed multivariate models using set 1, and the combinations of sets 1 and 2, and of sets 1, 2, and 3.

We constructed the multivariate prediction model in two phases. In the first phase, the variables that contributed most to improving the area under the ROC curve {Obuchowski, 2005 supra} were added to the model in a stepwise fashion, until the increase in the area under the ROC curve when changing the predictor variable set of the model equaled zero. In the second phase, the constructed model was cross-validated using 10-fold venetian blind crossvalidation, and the variable that showed most variation in the regression vector was removed through backward elemination. After the removal of a variable, the cross-validation was repeated. Variable removal was stopped

Table 6. Variables included in final multivariate models.

| Classical markers | Classical + NMR lipoprotein profile markers | Classical + NMR lipoprotein profile markers + lipoprotein metabolic ratios (1) | Classical + IVMR lipoprotein profile markers + lipoprotein metabolic ratios (2) |
|---|---|---|---|
| Age | Age | Age | Age |
| Cigarettes per day | | | Cigarettes per day |
| | Smokes & inhales | Smokes & inhales | |
| Total cholesterol | | | |
| | LDL particle number | LDL particle number | LDL particle number |
| Diastolic blood pressure (nurse) | Diastolic blood pressure (nurse) | Diastolic blood pressure (nurse) | Diastolic blood pressure (nurse) |
| HDL cholesterol | | | |
| | HDL particle number | HDL particle number | HDL particle number |
| Glucose | Glucose | | Glucose |
| Sex | Sex | Sex | Sex |
| Blood pressure medication | Blood pressure medication | Blood pressure medication | Blood Pressure medication |
| | | $\ln\left(\dfrac{J_{prod,TOT}}{\overline{k^{TOT}_{u,liver}}}\right)$ | $\ln\left(\dfrac{J_{prod,TOT}}{\overline{k^{TOT}_{u,liver}}}\right)$ |
| | | $\ln\left(\dfrac{J_{in,ILDL}}{\overline{k^{ILDL}_{a,liver}}}\right)$ | $\ln\left(\dfrac{J_{in,ILDL}}{\overline{k^{ILDL}_{a,liver}}}\right)$ |
| | | $\ln\left(\dfrac{J_{in,ILDL}}{\overline{k^{ILDL}_{l}}}\right)$ | $\ln\left(\dfrac{J_{in,ILDL}}{\overline{k^{ILDL}_{l}}}\right)$ |
| | | $\ln\left(\dfrac{\overline{k^{ILDL}_{l}}}{\overline{k^{ILDL}_{u,liver}}}\right)$ | $\ln\left(\dfrac{\overline{k^{ILDL}_{l}}}{\overline{k^{ILDL}_{u,liver}}}\right)$ |
| | | $\ln\left(\dfrac{\overline{k^{ILDL}_{a,liver}}}{\overline{k^{ILDL}_{l}}}\right)$ | $\ln\left(\dfrac{\overline{k^{ILDL}_{a,liver}}}{\overline{k^{ILDL}_{l}}}\right)$ |
| | | | $\ln\left(\dfrac{\overline{k^{ILDL}_{hl}}}{\overline{k^{ILDL}_{l}}}\right)$ |

when all variables were stable within the model (95% confidence interval of the regression coefficient did not pass through zero) or when removing more variables would amount to completely eliminating a classical risk factor from the model (for instance, there would be no variables with blood pressure information left).

For the combined sets, two alternatives for the first phase were used: one started the stepwise procedure from scratch, and the other started the stepwise procedure from the final cross-validated model from set 1. All analyses were carried out in Matlab version 7.7.0 with statistics toolbox version 7.0 and PLS toolbox version 5.0.3.

The multivariate predictions of CVD risk were compared using area under the cross-validated ROC curve statistics, using the method by de Long {DeLong, 1988 1021 /id} and a binomial exact test, calculated in MedCalc, version 11.4.4.0.

## RESULTS

### Baseline Characteristics

Of the 2142 subjects in the general research use assent group in which NMR lipoprotein profiles were measured with no previous cardiovascular disease, 145 cases and 1836 controls were found to have a complete record of all parameters to be included in the analysis. Baseline characteristics of the subjects are shown in Table 7. The mean age was 49±9 years, 52% was female.

### Multivariate Models

The variables included in the final multivariate models are shown in Table 6.

Table 7. Baseline characteristics of the subjects. *

| Characteristic | Men (N=946) | Women (N=1035) |
|---|---|---|
| Mean age - yr | 49.2±9.3 | 49.5±9.0 |
| Cholesterol - mg/dl | | |
|     total | 204±36 | 205±40 |
|     HDL | 44±11 | 56±15 |
| Blood pressure - mm Hg | | |
|     Systolic (nurse) | 127±16 | 122±19 |
|     Diastolic (nurse) | 80±10 | 75±10 |
| Blood pressure medication - no. (%) | 126 (13.3) | 128 (12.4) |
| Body-mass index | 27.6±3.8 | 25.8±5.1 |
| Smoking | | |
|     Smokes and inhales | 201 (21.2) | 219 (21.2) |
|     Cigarettes per day | 5.2±12.1 | 4.2±9.6 |
|     Smokes cigars - no. (%) | 46 (4.9) | 2 (0.2) |
|     Smokes pipe - no. (%) | 28 (3.0) | 0 (0) |
|     Spouse smokes - no. (%) | 344 (36.4) | 450 (43.5) |
| Glucose - mg/dl | 95±18 | 91±22 |

* Plus-minus values are means ± SD. To convert the values for cholesterol to millimoles per liter, multiply by 0.02568. The body-mass index is the weight in kilograms divided by the square of the height in meters. HDL denotes high-density lipoprotein.

Table 8. Statistical analysis of area under the ROC curve for the cross-validated multivariate models.

| Model 1 | Model 2 | Difference AUC ROC curve | Standard Error | P value |
|---|---|---|---|---|
| Classic | Classic & NMR | 0.00527 | 0.00583 | 0.3658 |
| Classic | Classic & NMR & ratios (1) | 0.0171 | 0.00851 | 0.0451 |
| Classic | Classic & NMR & ratios (2) | 0.0235 | 0.00851 | 0.0057 |
| Classic & NMR | Classic & NMR & ratios (1) | 0.0118 | 0.00657 | 0.0731 |

(continued)

| Model 1 | Model 2 | Difference AUC ROC curve | Standard Error | P value |
|---------|---------|--------------------------|----------------|---------|
| Classic & NMR | Classic & NMR & ratios (2) | 0.0182 | 0.00826 | 0.0272 |

**ROC analysis of Multivariate Models**

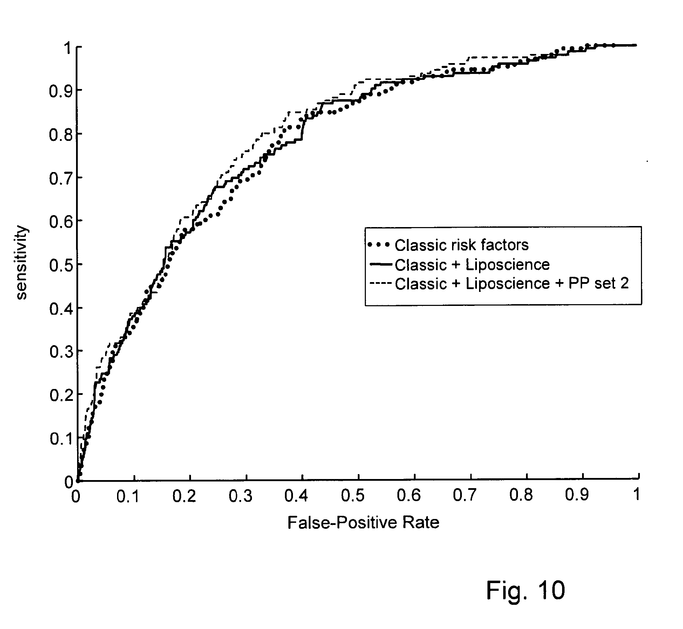

Receiver-Operating-Characteristic (ROC) curves for general cardiovascular disease are shown in Figure 10. Results of the statistical analyses comparing the curves are shown in Table 8. Adding Particle Profiler lipoprotein metabolic ratios (liporatios) to a model including existing cardiovascular risk factors significantly improved the area under the ROC curve for this population.

**Example 5**

Lipoprotein metabolic ratios improve diabetes risk prediction.

**Study Sample and Risk Factors**

In this study we used subjects from the Offspring cohort in the Framingham Heart Study (FHS) {Cromwell WC et al. J Clin Lipidology 2007;1(6):583-592}. Subjects were included when they had no history of diabetes, gave written informed consent for general research use, had complete NMR lipoprotein profiles recorded, and had a complete record of classical diabetes risk factors. Subjects were excluded when no diabetes data was available on the measuring day, or on any of the three earlier exams, or on the last examination day (3rd examination after lipoprotein measure, approx. 9-10 years later).

**Computational Modeling**

We applied the Particle Profiler computational model to the NMR lipoprotein profiles. Details can be found in the corresponding section of example 4.

**Outcomes**

Subjects who were recorded as diabetic by Framingham criteria in one of the three examinations following the lipoprotein profile measurement, but not before or at the time of the measurement, were designated as 'cases', subjects that did not develop diabetes at any time as 'controls'.

**Statistical Analysis**

We used multivariate logistic regression to correlate predictor variables with the CVD outcome. We grouped predictor variables into three sets: 1. classical cardiovascular risk parameters, 2. direct lipoprotein profile-based parameters, and 3. Particle Profiler-based parameters (See Table 9). We constructed multivariate models using set 1, and the combinations of sets 1 and 2, and sets 1, 2, and 3. For the combined sets, two models were constructed; one by starting to add variables from scratch and another by starting to add variables to the finished model constructed using set 1.

Table 9. Variables included in final multivariate models.

| Classical markers | Classical + NMR lipoprotein profile markers | Classical + NMR lipoprotein profile markers + lipoprotein metabolic ratios (1) | Classical + NMR lipoprotein profile markers + lipoprotein metabolic ratios (2) |
|---|---|---|---|
| Glucose | Glucose | Glucose | Glucose |
| BMI | BMI | BMI | BMI |
| Spouse smokes | Spouse smokes | Spouse smokes | Spouse smokes |
| Sex | Sex | Sex | Sex |

(continued)

| Spouse smokes | Spouse smokes | Spouse smokes | Spouse smokes |
|---|---|---|---|
| HDL cholesterol | HDL cholesterol | HDL cholesterol, | HDL cholesterol |
| Diastolic blood pressure (physician) | Diastolic blood pressure (physician) | Diastolic blood pressure (physician) | Diastolic blood pressure (physician) |
| | HDL particle number | HDL particle number | HDL particle number |
| | | VLDL size | |
| | VLDL particle number | | VLDL particle number |
| | | $\ln\left(\dfrac{\overline{J_{in,ILDL}}}{\overline{k^{ILDL}_{u,\,liver}}}\right)$ | $\ln\left(\dfrac{\overline{J_{in,ILDL}}}{\overline{k^{ILDL}_{u,\,liver}}}\right)$ |
| | | $\ln\left(\dfrac{\overline{k^{ILDL}_{lpl}}}{\overline{k^{ILDL}_{a,\,liver}}}\right)$ | $\ln\left(\dfrac{\overline{k^{ILDL}_{lpl}}}{\overline{k^{ILDL}_{a,\,liver}}}\right)$ |
| | | $\ln\left(\dfrac{\overline{k^{ILDL}_{hl}}}{\overline{k^{ILDL}_{l}}}\right)$ | $\ln\left(\dfrac{\overline{k^{ILDL}_{hl}}}{\overline{k^{ILDL}_{l}}}\right)$ |

We constructed the multivariate prediction model in two phases. In the first phase, the variables that contributed most to improving the area under the ROC curve (Obuchowski, *supra)* were added to the model in a stepwise fashion, until the increase in the area under the ROC curve when changing the predictor variable set of the model equaled zero. In the second phase, the constructed model was cross-validated using 10-fold venetian blind cross-validation, and the variable that showed most variation in the regression vector was removed through backward elemination. After the removal of a variable, the cross-validation was repeated. Variable removal was stopped when all variables were stable within the model (95% confidence interval of the regression coefficient did not pass through zero) or when removing more variables would amount to completely eliminating a classical risk factor from the model (for instance, there would be no variables with blood pressure information left).

For the combined sets, two alternatives for the first phase were used: one started the stepwise procedure from scratch, and the other started the stepwise procedure from the final cross-validated model from set 1. All analyses were carried out in Matlab version 7.7.0 with statistics toolbox version 7.0 and PLS toolbox version 5.0.3.

The multivariate predictions of diabetes risk were compared using area under the cross-validated ROC curve statistics, using the method by de Long (DeLong, ER et al, 1988, Biometrics 44:837-845) and a binomial exact test, calculated in MedCalc, version 11.4.4.0.

**RESULTS**

**Baseline Characteristics**

Of the 2142 subjects in the general research use assent group in which NMR lipoprotein profiles were measured with no previous diabetes, 145 cases and 1836 controls were found to have a complete record of all parameters to be included in the analysis. Baseline characteristics of the subjects are shown in Table 10. The mean age was 49t9 years, 52% was female.

Table 10. Baseline characteristics of the subjects. *

| Characteristic | Men (N=946) | Women (N=1035) |
|---|---|---|
| Mean aqe - yr | 49.4±9.2 | 49.7±9.0 |
| Cholesterol - mg/dl | | |

(continued)

| Characteristic | Men (N=946) | Women (N=1035) |
|---|---|---|
| total | 204±36 | 204±39 |
| HDL | 44±11 | 57±15 |
| Blood pressure - mm Hg | | |
| Systolic (nurse) | 127±16 | 121±18 |
| Diastolic (nurse) | 80±10 | 75±10 |
| Blood pressure medication - no. (%) | 131 (14.2) | 122 (12.4) |
| Bodv-mass index | 27.5±3.7 | 25.7±4.9 |
| Smoking | | |
| Smokes and inhales | 200 (21.8) | 204(20.8) |
| Cigarettes per day | 5.4±12.3 | 4.1±9.4 |
| Smokes cigars - no. (%) | 34 (3.7) | 2 (0.2) |
| Smokes pipe - no. (%) | 10 (1.1) | 0 (0) |
| Spouse smokes - no. (%) | 158 (17.2) | 173 (17.6) |
| Glucose - mg/dl | 92±9 | 89±9 |
| * Plus-minus values are means ± SD. To convert the values for cholesterol to millimoles per liter, multiply by 0.02568. The body-mass index is the weight in kilograms divided by the square of the height in meters. HDL denotes high-density lipoprotein. | | |

**Multivariate Models**

The variables included in the final multivariate models are shown in Table 9.

**ROC analysis of Multivariate Models**

Receiver-Operating-Characteristic (ROC) curves for diabetes development are shown in Figure 16. Results of the statistical analyses comparing the curves are shown in Table 11. Adding Particle Profiler parameters to a model including existing diabetes risk factors significantly improved the area under the ROC curve for this population.

Table 11. Statistical analysis of area under the ROC curve for the cross-validated multivariate models predicting diabetes development.

| Model 1 | Model 2 | Difference AUC ROC curve | Standard Error | P value |
|---|---|---|---|---|
| Classic | Classic & NMR | 0.00577 | 0.00337 | 0.0874 |
| Classic | Classic & NMR & ratios (1) | 0.0171 | 0.00669 | 0.0105 |
| Classic | Classic & NMR & ratios (2) | 0.0159 | 0.00640 | 0.0131 |
| Classic & NMR | Classic & NMR & ratios (1) | 0.0113 | 0.00566 | 0.0451 |
| Classic & NMR | Classic & NMR & ratios (2) | 0.0101 | 0.00527 | 0.0549 |

**Claims**

1.  Method to improve the risk analysis of a subject for developing a cardiovascular disease or a (pre-)diabetic condition, comprising including in the risk analysis lipoprotein metabolic ratios derived from the lipoprotein profile of said subject.

2.  Method according to claim 1, wherein the risk analysis is performed via a multivariate analysis.

3.  Method according to claim 1 or 2, where the disease is a cardiovascular disease, preferably a disease selected from the group of atrial fibrillation, (congestive) heart failure, coronary heart disease, intermittent claudication, stenosis, thromboembolism, myocardial infarction, coronary insufficiency, angina, ischemic stroke, hemorrhagic stroke, ischemia, and peripheral artery disease.

4.  Method according to claim 3, wherein the risk analysis is based on one or more of the classic parameters age of the subject, sex of the subject, systolic and/or diastolic blood pressure, body-mass index, smoking behaviour, serum cholesterol concentration and serum glucose concentration.

5.  Method according to claim 2, wherein the lipoprotein metabolic ratios from the lipoprotein profile included in the risk analysis are selected
    from the group of:

    $$\ln\left(\frac{J_{prod,TOT}}{k_{u,liver}^{TOT}}\right)$$

    or parameters that are highly correlated therewith as indicated in Table 2;

    $$\ln\left(\frac{J_{in,ILDL}}{k_{a,liver}^{ILDL}}\right)$$

    or parameters that are

    highly correlated therewith as indicated in Table 2; $\ln\left(\frac{J_{in,ILDL}}{k_1^{ILDL}}\right)$

    or

    parameters that are highly correlated therewith as indicated in Table 2; $\ln\left(\frac{\overline{k_1^{ILDL}}}{\overline{k_{u,liver}^{ILDL}}}\right)$

    or parameters that are highly correlated therewith as indicated in Table 2;

    $$\ln\left(\frac{\overline{k_{a,liver}^{ILDL}}}{\overline{k_1^{ILDL}}}\right)$$

    or parameters that are highly correlated therewith as

    indicated in Table 2; or $\ln\left(\frac{\overline{k_{hl}^{ILDL}}}{\overline{k_1^{ILDL}}}\right)$

    or parameters that are highly correlated therewith as indicated in Table 2; and any combinations thereof.

6.  Method according to claim 5, wherein at least two, preferably at least three, more preferably at least four and most preferably at least five of the lipoprotein metabolic ratios are included in the risk analysis.

7.  Method according to claim 1 or 2, where the disease is a (pre-)diabetic condition, preferably a disease selected from the group of diabetes, in particular diabetes type 2, insulin resistance, impaired glucose tolerance, metabolic syndrome, and obesitas.

8. Method according to claim 7, wherein the risk analysis is based on one or more of the classic parameters: sex of the subject, systolic and/or diastolic blood pressure, body-mass index, smoking behaviour of the spouse, serum HDL cholesterol concentration and serum glucose concentration and, optional, the HDL particle number and the VDL particle number.

9. Method according to claim 7 or 8, wherein the lipoprotein metabolic ratios from the lipoprotein profile included in the risk analysis are selected

from the group of: $\ln\left(\dfrac{J_{in,ILDL}}{k_{u,liver}^{ILDL}}\right)$

or parameters that are highly correlated

therewith as indicated in Table 3; $\ln\left(\dfrac{\overline{k_{lpl}^{ILDL}}}{k_{a,liver}^{ILDL}}\right)$

or parameters that are highly

correlated therewith as indicated in Table 3; $\ln\left(\dfrac{\overline{k_{hl}^{ILDL}}}{k_{l}^{ILDL}}\right)$

or parameters that
are highly correlated therewith as indicated in Table 3; and any combinations thereof.

10. Method according to any of claims 1 - 9, wherein the subject is a human being.

11. Use of lipoprotein metabolic rations from the lipoprotein profile of a subject for improving the risk analysis of a subject for developing a cardiovascular disease or a (pre-)diabetic condition.

Fig. 1

Fig. 2

Fig. 3

Fig. 4

Fig. 5

Fig. 6

Fig. 7

Fig. 8

Fig. 9

Fig. 10

Fig. 11

Fig. 12

EP 2 503 339 A1

Fig. 13A

Fig. 13B

EP 2 503 339 A1

Fig. 14

Fig. 15

Fig. 16

European Patent Office
Europäisches Patentamt
European Patent Office
Office européen des brevets

**EUROPEAN SEARCH REPORT**

Application Number

EP 11 15 9462

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHAN DICK C ET AL: "Relationships between cholesterol homoeostasis and triacylglycerol-rich lipoprotein remnant metabolism in the metabolic syndrome", CLINICAL SCIENCE, BIOCHEMICAL SOCIETY AND THE MEDICAL RESEARCH SOCIETY, LONDON, GB, vol. 104, no. 4, 1 April 2003 (2003-04-01), pages 383-388, XP008140517, ISSN: 0143-5221 * abstract * * page 387, column 2, paragraph 2 * | 1-11 | INV. G01N33/68 G01N33/92 |
| X | TER AVEST ET AL: "Remnant particles are the major determinant of an increased intima media thickness in patients with familial combined hyperlipidemia (FCH)", ATHEROSCLEROSIS, ELSEVIER IRELAND LTD, IE, vol. 191, no. 1, 13 February 2007 (2007-02-13), pages 220-226, XP005886403, ISSN: 0021-9150, DOI: 10.1016/J.ATHEROSCLEROSIS.2006.03.025 * abstract * | 1-11 | |
| X | US 2006/073097 A1 (MA YUANHONG [US] ET AL) 6 April 2006 (2006-04-06) * abstract; claim 1 * | 1-11 | TECHNICAL FIELDS SEARCHED (IPC) G01N |
| X | US 6 869 802 B1 (SCHWERTNER HARVEY A [US] ET AL) 22 March 2005 (2005-03-22) * abstract; claim 1 * | 1-11 | |
| X | WO 98/01759 A1 (FORBES MEDI TECH INC [CA]) 15 January 1998 (1998-01-15) * abstract; claims 1-3 * | 1-11 | |

The present search report has been drawn up for all claims

| Place of search | Date of completion of the search | Examiner |
|---|---|---|
| The Hague | 8 August 2011 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

**ANNEX TO THE EUROPEAN SEARCH REPORT
ON EUROPEAN PATENT APPLICATION NO.**

EP 11 15 9462

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2011

| Patent document cited in search report | | Publication date | Patent family member(s) | | Publication date |
|---|---|---|---|---|---|
| US 2006073097 | A1 | 06-04-2006 | NONE | | |
| US 6869802 | B1 | 22-03-2005 | NONE | | |
| WO 9801759 | A1 | 15-01-1998 | AU | 3251897 A | 02-02-1998 |
| | | | CA | 2259519 A1 | 15-01-1998 |
| | | | EP | 0912900 A1 | 06-05-1999 |
| | | | HU | 9904042 A2 | 28-03-2000 |
| | | | PL | 331006 A1 | 21-06-1999 |
| | | | US | 5965449 A | 12-10-1999 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009151324 A

**Non-patent literature cited in the description**

- **CROMWELL, W.C. ; OTVOS, J.D.** *Curr. Atheroscl. Rep.,* 2004, vol. 6, 381-387
- **GRUNDY, S.M. et al.** *Circulation,* 2004, vol. 110, 227-239
- **CAULFIELD, M. et al.** *Clin. Chem.,* 2008, vol. 54, 2088-2089
- **OLIVECRONA, T. et al.** *Proc. Nutr. Soc.,* 1997, vol. 56, 723-729
- **DEMANT, T. et al.** *J. Lipid Res.,* 1988, vol. 29, 1603-1611
- **PARHOFER, K. ; BARRETT, P.** *J. Lipid Res.,* 2006, vol. 47, 1620-1630
- **VAN SCHALKWIJK, D. et al.** *J. Lipid Res.,* 2009, vol. 50, 2398-2411
- **BILZ et al.** *J. Lipid Res.,* 2004, vol. 45, 174-185
- **FORSTER et al.** *Atherosclerosis,* 2002, vol. 164, 129-145
- **DEMANT, T.** *J. Clin. Invest.,* 1991, vol. 88, 1490-1501
- **WILSON et al.** *Circulation,* 1998, vol. 97, 1837-1847
- **WANG et al.** *JAMA,* 2003, vol. 290, 1049-1056
- **D'AGOSTINO et al.** *Stroke,* 1994, vol. 25 (1), 40-43
- **D'AGOSTINO et al.** *Am. Heart J.,* 2000, vol. 139 (2 I), 272-281
- **D'AGOSTINO.** *Circulation,* 2008, vol. 118 (4), e86
- **PACKARD et al.** *J. Lipid Res.,* 2000, vol. 41, 305-318
- **DEMANT et al.** *J. Lipid Res.,* 1993, vol. 34, 147-156

- *J. Clin. Invest.,* 1991, vol. 88, 1490-1501
- **PACKARD, C.J. et al.** *J. Lipid Res.,* 2000, vol. 41, 305-318
- **DEMANT, T. et al.** *J. Lipid Res.,* 1993, vol. 34, 147-156
- **DEMAND, T. et al.** *J. Clin. Invest.,* 1991, vol. 88, 1490-1501
- **PACKARD, C. et al.** *J. Lipid Res.,* 2000, vol. 41, 305-318
- **JAMES, R. et al.** *J. Lipid Res.,* 1989, vol. 30, 159-169
- **DEMAND, T. et al.** *Am. J. Physiol.,* 1996, vol. 270, E 1022-E 1036
- **DEMAND, T. et al.** *Kidney Int.,* 1998, vol. 54, 2064-2080
- **LUNDAHL, B. et al.** *Am. J. Physiol. Endocrinol. Metab.,* 2006, vol. 290, E739-E745
- **SCHMITZ, M. et al.** *J. Acquir. Immune Defic. Syndr.,* 2001, vol. 26, 225-235
- **BILZ, S. et al.** *J. Lipids Res.,* 2004, vol. 45, 174-185
- **FORSTER, L. et al.** *Atherosclerosis,* 2002, vol. 164, 129-145
- **PACKARD, C. et al.** *J. Clin. Endocrinol. Metab.,* 1993, vol. 76, 1209-1216
- **AUSTIN, M. et al.** *Circulation,* 1990, vol. 82, 495-506
- **OBUCHOWSKI, N.** *AJR Am. J. Roentgenol.,* 2005, vol. 184, 364-372
- **CROMWELL WC et al.** *J Clin Lipidology,* 2007, vol. 1 (6), 583-592
- **DELONG, ER et al.** *Biometrics,* 1988, vol. 44, 837-845